# EUROPÄISCHE PATENTSCHRIFT

(11) **EP 1 117 645 B1**
(45) Veröffentlichungstag und Bekanntmachung des Hinweises auf die Patenterteilung: **06.05.2004**
(21) Anmeldenummer: 99970083.4
(22) Anmeldetag: 18.09.1999
(51) Int. Cl.: C07D 213/74, A61K 31/44

(54) **ARYLSUBSTITUIERTE PROPANOLAMINDERIVATE, VERFAHREN ZU DEREN HERSTELLUNG, DIESE VERBINDUNGEN ENTHALTENDE ARZNEIMITTEL UND DEREN VERWENDUNG**
ARYL-SUBSTITUTED PROPANOLAMINE DERIVATIVES, METHODS FOR THEIR PRODUCTION, MEDICAMENTS CONTAINING SAID COMPOUNDS AND THEIR USE
DERIVES DE PROPANOLAMINE SUBSTITUES PAR ARYLE, PROCEDES PERMETTANT DE LES PREPARER, MEDICAMENTS CONTENANT LESDITS COMPOSES ET LEUR UTILISATION

(30) Priorität: 02.10.1998 DE 19845405
(43) Veröffentlichungstag der Anmeldung: 25.07.2001
(73) Patentinhaber: Aventis Pharma Deutschland GmbH, 65929 Frankfurt am Main (DE)
(72) Erfinder: FRICK, Wendelin, D-65510 Hünstetten-Beuerbach (DE); KIRSCH, Reinhard, D-38100 Braunschweig (DE); GLOMBIK, Heiner, D-65719 Hofheim (DE); KRAMER, Werner, D-55130 Mainz-Laubenheim (DE); SCHÄFER, Hans-Ludwig, D-65239 Hochheim (DE)
(86) Internationale Anmeldenummer: PCT/EP1999/006931
(87) Internationale Veröffentlichungsnummer: WO 2000/020392

(56) Entgegenhaltungen:
- EP-A- 0 557 879
- EP-A- 0 869 121
- WO-A-93/16055

## Beschreibung

Die Erfindung betrifft substituierte Propanolaminderivate und deren Säureadditionssalze.

Es sind bereits mehrere Wirkstoffklassen zur Behandlung von Adipositas und von Lipidstoffwechselstörungen beschrieben worden:
- polymere Adsorber, wie z.B. Cholestyramin
- Benzothiazepine (WO 93/16055)
- Gallensäuredimere und -konjugate (EP 0 489 423)
- 4-Amino-2-ureido-pyrimidin-5-carbonsäureamide (EP 0 557 879)

Der Erfindung lag die Aufgabe zugrunde, weitere Verbindungen zur Verfügung zu stellen, die eine therapeutisch verwertbare hypolipidämische Wirkung entfalten. Die Erfindung betrifft daher Propanolaminderivate der Formel I, 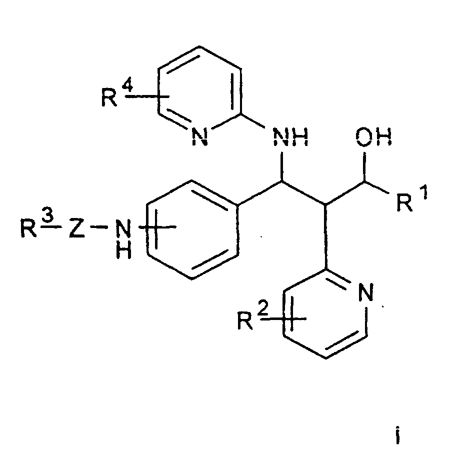 worin bedeuten
- R¹: Phenyl, Heteroaryl, unsubstituiert oder gegebenenfalls mit ein bis drei voneinander unabhängigen Resten substituiert wobei der Aromat oder Heteroaromat ein bis dreifach substituiert sein kann mit Fluor, Chlor, Brom, Jod, OH, CF₃, -NO₂, CN, (C₁-C₈)-Alkoxy, (C₁-C₈)-Alkyl, NH₂, -NH-R⁹, -N(R⁹)R¹⁰, CHO, -COOH, -COOR¹¹, -(C=O)-R¹², (C₁-C₆)-alkyl-OH, (C₁-C₆)-alkyl(-OH)-Phenyl, (C₁-C₆)-alkyl-CF₃, (C₁-C₆)-alkyl-NO₂, (C₁-C₆)-alkyl-CN, (C₁-C₆)-alkyl-NH₂, (C₁-C₆)-alkyl-NH-R⁹, (C₁-C₆)-alkyl-N(R⁹)R¹⁰, (C₁-C₆)-alkyl-CHO, (C₁-C₆)-alkyl-COOH, (C₁-C₆)-alkyl-COOR¹¹, (C₁-C₆)-alkyl-(C=O)-R¹², -O-(C₁-C₆)-alkyl-OH, -O-(C₁-C₆)-alkyl-CF₃, -O-(C₁-C₆)-alkyl-NO₂, -O-(C₁-C₆)-alkyl-CN, -O-(C₁-C₆)-alkyl-NH₂, -O-(C₁-C₆)-alkyl-NH-R⁹, -O-(C₁-C₆)-alkyl-N(R⁹)R¹⁰, -O-(C₁-C₆)-alkyl-CHO, -O-(C₁-C₆)-alkyl-COOH, -O-(C₁-C₆)-alkyl-COOR¹¹, -O-(C₁-C₆)-alkyl-(C=O)-R¹², -N-SO₃H, -SO₂-CH₃, -O-(C₁-C₆)-alkyl-O-(C₁-C₆)-alkyl-Phenyl, , (C₁-C₆)-Alkylthio, Pyridyl, wobei in den Alkylresten ein oder mehrere Wasserstoff(e) durch Fluor ersetzt sein können und wobei Phenyl und Pyridyl ihrerseits einfach durch Methyl, Methoxy oder Halogen substituiert sein können;
- R²: H, OH, CH₂OH, OMe, CHO, NH₂;
- R³: Zuckerrest, Dizuckerrest, Trizuckerrest, Tetrazuckerrest, wobei der Zuckerrest, Dizuckerrest, Trizuckerrest oder Tetrazuckerrest gegebenenfalls ein oder mehrfach substituiert ist durch eine der Zucker-Schutzgruppen, HO-SO₂-, (HO)₂-PO-;
- R⁴: H, Methyl, F, OMe;
- R⁹ bis R¹²: unabhängig voneinander H, C₁-C₈-Alkyl;
- Z: - NH-C₀-C₁₆-Alkyl-C=O -, -O-C₀-C₁₆-Alkyl-C=O -,
-(C=O)ₘ-C₁-C₁₆-Alkyl-(C=O)ₙ, Aminosäurerest, Diaminosäurerest, wobei der Aminosäurerest oder Diaminosäurerest gegebenenfalls ein oder mehrfach substituiert ist durch eine Aminosäure-Schutzgruppe, eine kovalente Bindung;
- n: 0 oder 1;
- m: 0 oder 1;
sowie deren pharmazeutisch verträglichen Salze und Ester.

Bevorzugt sind Verbindungen der Formel I, in denen ein oder mehrere Rest(e) die folgende Bedeutung hat bzw. haben:
- R¹: Phenyl, Pyridyl, Thienyl, Furyl, Pyrimidyl, Indolyl, Thiazolyl, Imidazolyl, Coumarinyl, Phthaliminyl, Chinoyl, Piperazinyl, Tetrazolyl, Triazolyl, Oxazolyl Isoxazolyl, Isthiazolyl oder deren benzoannelierte Derivate, wobei der Aromat oder Heteroaromat ein bis dreifach substituiert sein kann mit Fluor, Chlor, Brom, Jod, OH, CF₃, -NO₂, CN, (C₁-C₈)-Alkoxy, (C₁-C₈)-Alkyl, NH₂, -NH-R⁹, -N(R⁹)R¹⁰, CHO, -COOH, -COOR¹¹, -(C=O)-R¹², (C₁-C₆)-alkyl-OH, (C₁-C₆)-alkyl(-OH)-Phenyl, (C₁₋C₆)-alkyl-CF₃, (C₁-C₆)-alkyl-NO₂, (C₁-C₆)-alkyl-CN, (C₁-C₆)-alkyl-NH₂, (C₁-C₆)-alkyl-NH-R⁹, (C₁-C₆)-alkyl-N(R⁹)R¹⁰, (C₁-C₆)-alkyl-CHO, (C₁-C₆)-alkyl-COOH, (C₁-C₆)-alkyl-COOR¹¹, (C₁-C₆)-alkyl-(C=O)-R¹², -O-(C₁-C₆)-alkyl-OH, -O-(C₁-C₆)-alkyl-CF₃, -O-(C₁-C₆)-alkyl-NO₂, -O-(C₁-C₆)-alkyl-CN, -O-(C₁-C₆)-alkyl-NH₂, -O-(C₁-C₆)-alkyl-NH-R⁹, -O-(C₁-C₆)-alkyl-N(R⁹)R¹⁰, -O-(C₁-C₆)-alkyl-CHO, -O-(C₁-C₆)-alkyl-COOH, -O-(C₁-C₆)-alkyl-COOR¹¹, -O-(C₁-C₆)-alkyl-(C=O)-R¹², -N-SO₃H, -SO₂-CH₃, -O-(C₁-C₆)-alkyl-O-(C₁-C₆)-alkyl-Phenyl, , (C₁-C₆)-Alkylthio, Pyridyl, wobei in den Alkylresten ein oder mehrere Wasserstoff(e) durch Fluor ersetzt sein können und wobei Phenyl und Pyridyl ihrerseits einfach durch Methyl, Methoxy oder Halogen substituiert sein können;
- R²: H, OH, CH₂OH, OMe, CHO, NH₂;
- R³: Zuckerrest, Dizuckerrest, Trizuckerrest, Tetrazuckerrest, wobei der Zuckerrest, Dizuckerrest, Trizuckerrest oder Tetrazuckerrest gegebenenfalls ein oder mehrfach substituiert ist durch eine der Zucker-Schutzgruppen, HO-SO₂-, (HO)₂-PO-;
- R⁴: H, Methyl, F, OMe;
- R⁹ bis R¹²: unabhängig voneinander H, C₁-C₈-Alkyl
- Z: - NH-C₀-C₁₆-Alkyl-C=O -, -O-C₀-C₁₆-Alkyl-C=O -,
-(C=O)ₘ-C₁-C₁₆-Alkyl-(C=O)ₙ, Aminosäurerest, Diaminosäurerest,
wobei der Aminosäurerest oder Diaminosäurerest gegebenenfalls ein oder mehrfach substituiert ist durch eine Aminosäure-Schutzgruppe, eine kovalente Bindung;
- n: 0 oder 1;
- m: 0 oder 1;
sowie deren pharmazeutisch verträglichen Salze und Ester.

Besonders bevorzugt sind Verbindungen der Formel I, in denen ein oder mehrere Rest(e) die folgende Bedeutung hat bzw. haben:
- R¹: Phenyl, Pyridyl, Thienyl, Furyl, Pyrimidyl, Indolyl, Thiazolyl, Imidazolyl, Coumarinyl, Phthaliminyl, Chinoyl, Piperazinyl, Tetrazolyl, Triazolyl, Oxazolyl, Isoxazolyl, Isthiazolyl, wobei der Aromat oder Heteroaromat ein bis zweifach substituiert sein kann mit Fluor, Chlor, Brom, Jod, OH, CF₃, -NO₂, CN, (C₁-C₈)-Alkoxy, (C₁-C₈)-Alkyl, C₃-C₆-Cycloalkyl, NH₂, CHO, -COOH, OCF₃
- R²: H, OH, CH₂OH, OMe, CHO, NH₂;
- R³: Zuckerrest, Dizuckerrest, wobei der Zuckerrest oder Dizuckerrest gegebenenfalls ein oder mehrfach substituiert ist durch eine der Zucker-Schutzgruppen, HO-SO₂-, (HO)₂-PO-;
- R⁴: H, Methyl, F, OMe;
- Z: - NH-C₀-C₁₆-Alkyl-C=O -, -O-C₀-C₁₆-Alkyl-C=O -,
-(C=O)ₘ-C₁-C₁₆-Alkyl-(C=O)ₙ, eine kovalente Bindung;
- n: 0 oder 1;
- m: 0 oder 1;
sowie deren physiologisch verträglichen Säureadditionssalze.

Ganz besonders bevorzugt sind Verbindungen der Formel I, in denen ein oder mehrere Rest(e) die folgende Bedeutung hat bzw. haben:
- R¹: Phenyl, Thiazolyl, Oxazolyl, Isoxazolyl, wobei der Aromat oder Heteroaromat ein bis zweifach substituiert sein kann mit Fluor, Chlor, Brom, (C₁-C₈)-Alkyl;
- R²: H, OH, CH₂OH, OMe, CHO, NH₂;
- R³: 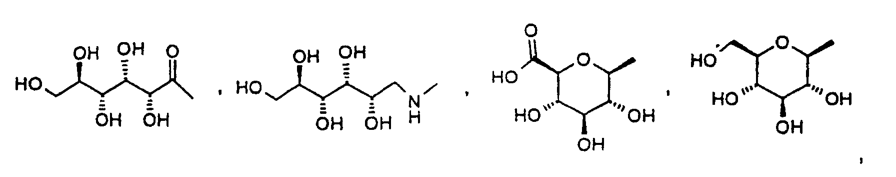 wobei der Zuckerrest gegebenenfalls ein oder mehrfach substituiert ist durch eine der Zucker-Schutzgruppen, HO-SO₂-;
- R⁴: H, Methyl, F, OMe;
- Z: - NH-C₆-C₁₂-Alkyl-C=O -, -O-C₆-C₁₂-Alkyl-C=O -,
-(C=O)ₘ-C₆-C₁₂-Alkyl-(C=O)ₙ;
- n: 0 oder 1;
- m: 0 oder 1;
sowie deren physiologisch verträglichen Säureadditionssalze.

In der oben genannten Heteroarylgruppen kommen als Heteroatome insbesondere z.B. O,S, N in Betracht.
Soweit nicht anders definiert, haben die heteroaromatischen Ringe 1-15 C-Atome und 1-6 Heteroatome, vorzugsweise 1-5 C-Atome und 1-2 Heteroatome.
Für die in den vorangegangenen Definitionen genannten Heteroarylgruppen kommen beispielsweise Thiophen, Furan, Pyridin, Pyrimidin, Indol, Chinolin, Oxazol, Isoxazol, Thiazol oder Isthiazol in Frage.

Unter dem Begriff Alkyl werden geradkettige oder verzweigte Kohlenwasserstoffketten verstanden.

Unter Zuckerresten werden Verbindungen verstanden, die sich von Aldosen und Ketosen mit 3 bis 7 Kohlenstoffatomen ableiten, die der D- oder L-Reihe angehören können; dazu gehören auch Aminozucker, Zuckeralkohole oder Zuckersäuren. Beispielhaft seien genannt Glucose, Mannose, Fructose, Galaktose, Ribose, Erythrose, Glycerinaldehyd, Sedoheptulose, Glucosamin, Galaktosamin, Glucuronsäure, Galakturonsäure, Gluconsäure, Galaktonsäure, Mannonsäure, Glucamin, 3-Amino-1,2-propandiol, Glucarsäure und Galaktarsäure.

Mit Dizucker sind Saccharide gemeint, die aus zwei Zuckereinheiten bestehen.
Di-, Tri-, oder Tetrasaccharide entstehen durch acetalartige Bindung von 2 oder mehreren Zuckern. Die Bindungen können dabei in der α- oder β-Form auftreten.
Beispielhaft seien genannt Laktose, Maltose und Cellobiose.

Wenn der Zucker substituiert ist, so erfolgt die Substitution bevorzugt am Wasserstoffatom einer OH-Gruppe des Zuckers.

Für die Hydroxygruppen der Zucker kommen im wesentlichen folgende Schutzgruppen in Frage: Benzyl-, Acetyl-, Benzoyl-, Pivaloyl-, Trityl-, tert.-Butyldimethylsilyl-, Benzyliden-, Cyclohexyliden- oder Isopropylidenschutzgruppen.

Mit dem Begriff Aminosäuren bzw. Aminosäurereste sind die stereoisomeren Formen, d.h. D- oder L-Formen, folgender Verbindungen gemeint:

| | | |
|---|---|---|
| Alanin | Glycin | Prolin |
| Cystein | Histidin | Glutamin |
| Asparaginsäure | Isoleucin | Arginin |
| Glutaminsäure | Lysin | Serin |
| Phenylalanin | Leucin | Threonin |
| Tryptophan | Methionin | Valin |
| Tyrosin | Asparagin | |

| | |
|---|---|
| 2-Aminoadipinsäure | 2-Aminoisobuttersäure |
| 3-Aminoadipinsäure | 3-Aminoisobuttersäure |
| beta-Alanin | 2-Aminopimelinsäure |
| 2-Aminobuttersäure | 2,4-Diaminobuttersäure |
| 4-Aminobuttersäure | Desmosin |
| Piperidinsäure | 2,2-Diaminopimelinsäure |
| 6-Aminocapronsäure | 2,3-Diaminopropionsäure |
| 2-Aminoheptansäure | N-Ethylglycin |
| 2-(2-Thienyl)-glycin | 3-(2-Thienyl)-alanin |
| Penicillamin | N-Methylglycin |
| N-Ethylasparagin | N-Methylisoleucin |
| Hydroxylysin | 6-N-Methyllysin |
| allo-Hydroxylysin | N-Methylvalin |
| 3-Hydroxyprolin | Norvalin |
| 4-Hydroxyprolin | Norleucin |
| Isodesmosin | Ornithin |
| allo-Isoleucin | 11-Aminoundecansäure |

Unter dem Begriff Aminosäureschutzgruppen sind geeignete Gruppen zu verstehen mit denen die funktionellen Gruppen der Seitenketten der Aminosäurereste geschützt sind (siehe z. B. T. W. Greene, P. G. M. Wuts, Protective Groups in Organic Synthesis, 2^{nd} Edition, John Wiley and Sons, New York 1991). Hauptsächlich wurden verwendet: t-butyloxy-carbonyl (BOC), 9-fluorenylmethoxycarbonyl (Fmoc), benzyloxy-carbonyl (Z), 2-(3,5-dimethoxyphenyl)prop-2-yloxycarbonyl (Ddz), methyl, t-butyl, trityl, S-t-butyl.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isothion-, Milch-, Lactobion-, Malein-, Apfel-, Methansulfon-, Bernstein-, p-Toluolsulfon-, Wein- und Trifluoressigsäure. Für medizinische Zwecke wird in besonders bevorzugter Weise das Chloridsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "Ester" im Zusammensetzung mit den Verbindungen der Formel I bezeichnet einen Ester dieser Verbindungen; der bei Verabreichung an einen Säuger, wie z.B. den Menschen, in der Lage ist, (direkt oder indirekt) eine Verbindung der Formel I zu bilden.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und Ester wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,1 mg bis 100 mg (typischerweise von 0,1 mg und 50 mg) pro Tag pro Kilogramm Körpergewicht, z.B. 0,1-10 mg/kg/Tag. Tabletten oder Kapseln, können beispielsweise von 0,01 bis 100 mg, typischerweise von 0,02 bis 50 mg enthalten. Im Falle pharmazeutisch verträglicher Salze beziehen sich die vorgenannten Gewichtsangaben auf das Gewicht des vom Salz abgeleiteten Aminopropanol-Ions. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säureund magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Gegenstand der Erfindung sind weiterhin sowohl Isomerengemische der Formel I, als auch die reinen Diastereomere der Formel I.

Die Erfindung betrifft weiterhin Verfahren zur Herstellung von Propanolaminderivaten der Formel I.

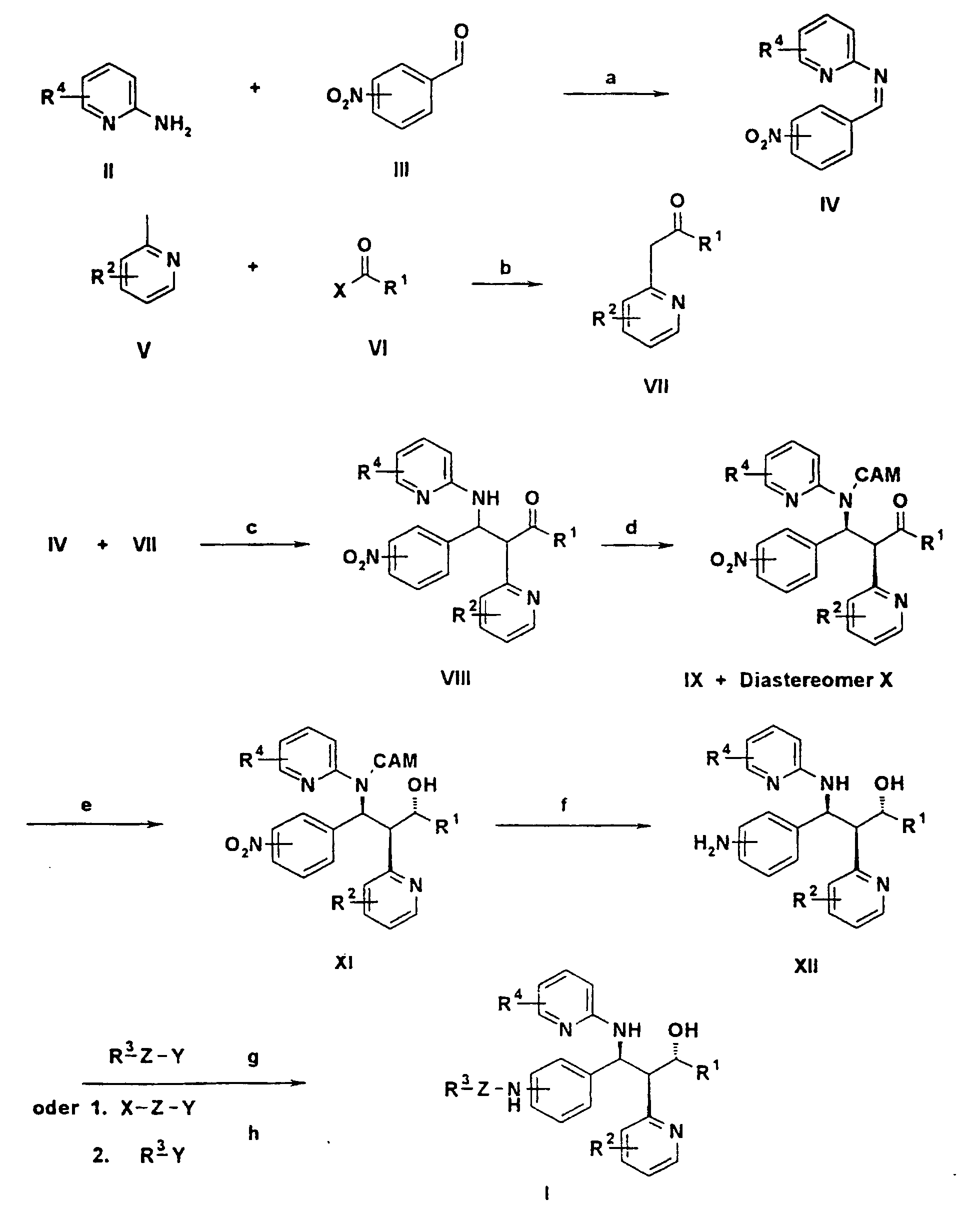

Verfahren zur Herstellung der Verbindungen der Formel !, dadurch gekennzeichnet, daß mit R⁴ substituierte Imine, wobei R⁴ die zu Formel I angegebene Bedeutung hat, aus Aminen des Typs II und Aldehyden des Typs III hergestellt werden. Hierzu werden zum Beispiel das Amin II und der Aldehyd III in Substanz oder in einem geeigneten Lösungsmittel wie Ethanol, Toluol oder Essigsäure ohne oder mit Zusatz einer Säure, z.B. p-Toluolsulfonsäure, bei Temperaturen von 20°-150°C zur Reaktion gebracht (a).

Mit Resten R¹ und R² substituierte Ketoverbindungen der Formel VII, wobei R¹ und R² die zu Formel I angegebene Bedeutung haben, werden nach literaturbekannten Verfahren oder in Anlehnung an solche Verfahren hergestellt. So werden beispielsweise Picolinderivate V mit geeigneten Base, wie n-Butyllithium, metalliert und in Tetrahydrofuran oder einem anderen geeigneten Lösungsmittel mit den entsprechenden Carbonsäurederivaten VI, z.B. als Carbonsäuredialkylamide oder - ester (Rest X) vorliegend bei Temperaturen zwischen -80° und 20°C umgesetzt (b).

Verbindungen des Typs VIII werden erhalten, indem Imine des Typs IV und Ketone des Typs VII, jeweils substituiert mit den Resten R¹, R² und R⁴, wobei R¹, R² und R⁴ die zu Formel I angegebene Bedeutung haben, zur Reaktion gebracht werden. Diese Reaktion kann beispielsweise durch Mischung der beiden Verbindungen in Substanz, ohne Lösungsmittel und anschließendem Erhitzen, oder in einem geeigneten Lösungsmittel wie Methylenchlorid, Ethanol, Toluol, Diglyme oder Tetradecan bei Temperaturen von 20°C bis 150°C durchgeführt werden (c).

Die racemischen Verbindungen des Typs VIII werden mit Hilfe einer chiralen Säure (z. B. mit Camphansäure, Penta-acetyl-gluconsäure, Campher-10-sulfonsäure, O-Methyl-mandelsäure oder Milchsäure) in die reinen Diastereomeren des Typs IX und X durch Kristallisation oder durch Chromatographie getrennt (d).

Die Ketoverbindungen des Typs IX oder X werden in einem geeigneten Lösungsmittel, wie z.B. Methanol, THF oder THF/Wasser mit NaBH₄ oder einem anderen geeigneten Reduktionsmittel bei Temperaturen zwischen -30° und +40°C zu Hydroxyverbindungen des Typs XI reduziert, wobei diese Verbindungen mit den Resten R¹, R² und R⁴ substituiert sein kann und R¹, R² und R⁴ die zu Formel I angegebene Bedeutung haben (e).

Die chirale Säure wird in einem geeignetem Lösungsmittel, wie z.B. Methanol, Ethanol, THF oder THF/Wasser unter basischen oder sauren Bedingungen, wie z. B. mit KOH, NaOH oder HCl, abgespalten. Anschließend wird die Nitrogruppe nach literaturbekannten Verfahren zum Amin reduziert und man erhält Verbindungen des Typs XII mit den Resten R¹, R² und R⁴ (f).

Die Aminoverbindungen des Typs XII werden mit den Alkyl- oder Acylresten R³-Z-Y, wobei Y eine Abgangsgruppe darstellt, mit literaturbekannten Methoden umgesetzt und man erhält Verbindungen der Formel I (g). Wenn man als Alkyl- oder Acylrest X-Z-Y einsetzt, wobei X eine Schutzgruppe darstellt, erhält man Zwischenverbindungen die mit weiteren Alkyl- oder Acylresten R³-Y zu Verbindungen der Formel I umgesetzt werden können (h).

Die Verbindungen der Formel I und deren pharmazeutisch verträgliche Salze und Ester stellen ideale Arzneimittel zur Behandlung von Lipidstoffwechselstörungen, insbesondere von Hyperlipidämie dar. Die Verbindungen der Formel I eignen sich ebenfalls zur Beeinflussung des Serumcholesterinspiegels sowie zur Prävention und Behandlung arteriosklerotischer Erscheinungen. Die Verbindungen können gegebenenfalls auch in Kombination mit Statinen, wie z.B. Simvastatatin, Fluvastatin, Pravastatin, Cerivastatin, Lovastatin oder Atorvastin verabreicht werden. Folgende Befunde belegen die pharmakologische Wirksamkeit der erfindungsgemäßen Verbindungen.

Die biologische Prüfung der erfindungsgemäßen Verbindungen erfolgte durch Ermittlung der Hemmung der [³H]-Taurocholataufnahme in Bürstensaummembranvesikel des Ileums von Kaninchen. Der Hemmtest wurde wie folgt durchgeführt:

### 1. Präparation von Bürstensaummembranvesikeln aus dem Ileum von Kaninchen

Die Präparation von Bürstensaummembranvesikeln aus den Darmzellen des Dünndarm erfolgte mit der sogenannten Mg²⁺-Präzipitationsmethode. Männliche Neuseeland-Kaninchen (2 bis 2,5 kg Körpergewicht) wurden durch intravenöse Injektion von 0,5 ml T61®, einer wäßrigen Lösung von 2,5 mg Tetracain HCl, 100 mg Embutramid und 25 mg Mebezoniumjodid getötet. Der Dünndarm wurde entnommen und mit eiskalter physiologischer Kochsalzlösung gespült. Die terminalen 7/10 des Dünndarms (gemessen in oral rektaler Richtung, d.h. das terminale Ileum, welches das aktive Na⁺-abhängige Gallensäuretransportsystem enthält) wurden zur Präparation der Bürstensaummembranvesikel verwendet. Die Därme wurden in Kunststoffbeuteln unter Stickstoff bei -80°C eingefroren. Zur Präparation der Membranvesikel wurden die eingefrorenen Därme bei 30°C im Wasserbad aufgetaut. Die Mucosa wurde abgeschabt und in 60 ml eiskaltem 12 mM Tris/HCl-Puffer (pH 7,1)/300 mM Mannit, 5 mM EGTA/10 mg/l Phenylmethyl-sulfonylfluorid/1 mg/l Trypsin Inhibitor v. Sojabohnen (32 (U/mg)/0,5 mg/l Trypsin Inhibitor v. Rinderlunge (193 U/mg)/5 mg/l Bacitracin suspendiert. Nach dem Verdünnen auf 300 ml mit eiskaltem destilliertem Wasser wurde mit einem Ultraturrax (18-Stab, IKA Werk Staufen, Deutschland) 3 Minuten bei 75 % max. Leistung unter Eiskühlung homogenisiert. Nach Zugabe von 3 ml 1 M MgCl₂-Lösung (Endkonzentration 10 mM) ließ man exakt 1 Minute bei 0°C stehen. Durch Zugabe von Mg²⁺ aggregieren die Zellmembranen und präzipitieren mit Ausnahme der Bürstensaummembranen. Nach einer 15-minütigen Zentrifugation bei 3000 x g (5000 rpm, SS-34-Rotor) wird der Niederschlag verworfen und der Überstand, der die Bürstensaummembranen enthält, 30 Minuten bei 48000 x g (20000 rpm, SS-34-Rotor) zentrifugiert. Der Überstand wurde verworfen, der Niederschlag in 60 ml 12 mM Tris/HCl-Puffer (pH 7,1)/60 mM Mannit, 5 mM EGTA mit einem Potter Elvejhem Homogenisator (Braun, Melsungen, 900 rpm, 10 Hübe) rehomogenisiert. Nach Zugabe von 0,1 ml 1 M MgCl₂-Lösung und 15-minütiger Inkubationszeit bei 0°C wurde erneut 15 Minuten bei 3000 x g zentrifugiert. Der Überstand wurde anschließend nochmals 30 Minuten bei 48000 x g (20000 rpm, SS-34-Rotor) zentrifugiert. Der Niederschlag wurde in 30 ml 10 mM Tris/Hepes-Puffer (pH 7,4)/300 mM Mannit aufgenommen und durch 20 Hübe in einem Potter Elvejhem Homogenisator bei 1000 rpm homogen resuspendiert. Nach 30 minütiger Zentrifugation bei 48000 x g (20000 rpm, SS-34-Rotor) wurde der Niederschlag in 0,5 bis 2 ml Tris/Hepes-Puffer (pH 7,4)/280 mM Mannit (Endkonzentration 20 mg/ml) aufgenommen und mit Hilfe einer Tuberkulinspritze mit einer 27 Gauge-Nadel resuspendiert. Die Vesikel wurden entweder unmittelbar nach der Präparation für Transportuntersuchungen verwendet oder bei -196°C in 4 mg Portionen in flüssigem Stickstoff aufbewahrt.

### 2. Hemmung der Na⁺-abhängigen [³H]Taurocholat-Aufnahme in Bürstensaummembranvesikel des Ileums

Die Aufnahme von Substraten in die vorstehend beschriebenen Bürstensaummembranvesikel wurde mittels der sogenannten Membranfiltrationstechnik bestimmt. 10 µl der Vesikelsuspension (100 µg Protein) wurden als Tropfen an die Wand eines Polystyrolinkubationsröhrchens (11 x 70 mm) pipettiert, welches das Inkubationsmedium mit den entsprechenden Liganden enthielt (90 µl). Das Inkubationsmedium enthielt 0,75 µl = 0,75 µCi [³H(G)]-Taurocholat (spezifische Aktivität: 2,1 Ci/mMol), /0,5 µl 10 mM Taurocholat/8,75 µl Natrium-Transport-Puffer (10 mM Tris/Hepes, (pH 7,4)/100 mM Mannit/100 mM NaCl) (Na-T-P) bzw. 8,75 µl Kalium-Transport-Puffer (10 mM Tris/Hepes (pH 7,4)/100 mM Mannit/100 mM KCl) (K-T-P) und 80 µl der betreffenden Inhibitorlösung, je nach Experiment in Na-T-Puffer bzw. K-T-Puffer gelöst. Das Inkubationsmedium wurde durch ein Polyvinylidenfluorid-Membranfilter (SYHV LO 4NS, 0,45 µm, 4 mm Ø, Millipore, Eschborn, Deutschland) filtriert. Durch Vermischung der Vesikel mit dem Inkubationsmedium wurde die Transportmessung gestartet. Die Konzentration an Taurocholat im Inkubationsansatz betrug 50 µM. Nach der gewünschten Inkubationszeit (üblicherweise 1 Minute) wurde der Transport durch Zugabe von 1 ml eiskalter Stoplösung (10 mM Tris/Hepes, (pH 7,4)/150 mM KCI) gestoppt. Die entstehende Mischung wurde sofort bei einem Vakuum von 25 bis 35 mbar über ein Membranfilter aus Cellulosenitrat (ME 25, 0,45 µm, 25 mm Durchmesser, Schleicher & Schuell, Dassell, Deutschland) abgesaugt. Der Filter wurde mit 5 ml eiskalter Stoplösung nachgewaschen.

Zur Messung der Aufnahme des radioaktiv markierten Taurocholats wurde das Membranfilter mit 4 ml des Szintillators Quickszint 361 (Zinsser Analytik GmbH, Frankfurt, Deutschland) aufgelöst und die Radioaktivität durch Flüssigkeits-Szintillationsmessung in einem Meßgerät TriCarb 2500 (Canberra Packard GmbH, Frankfurt, Deutschland) gemessen. Die gemessenen Werte wurden nach Eichung des Gerätes mit Hilfe von Standardproben und nach Korrektur evtl. vorhandener Chemilumineszenz als dpm (Decompositions per Minute) erhalten.

Die Kontrollwerte wurden jeweils in Na-T-P und K-T-P ermittelt. Die Differenz zwischen der Aufnahme in Na-T-P und K-T-P ergab den Na⁺-abhängigen Transportanteil. Als IC₅₀ Na⁺ wurde diejenige Konzentration an Inhibitor bezeichnet, bei der der Na⁺-abhängige Transportanteil um 50 % - bezogen auf die Kontrolle - gehemmt war.

Die pharmakologischen Daten umfassen eine Testserie, in der die Interaktion der erfindungsgemäßen Verbindungen mit dem intestinalen Gallensäuretransportsystem im terminalen Dünndarm untersucht wurde. Die Ergebnisse sind in Tabelle 1 zusammengefaßt.

Tabelle 1 zeigt Meßwerte (Biolog. Test) der Hemmung der [³H]-Taurocholataufnahme in Bürstensaummembranvesikel des Ileums von Kaninchen. Angegeben sind die Quotienten aus den IC_{50Na}-Werten der Referenzsubstanz als Taurochenodesoxycholat (TCDC) und der jeweiligen Testsubstanz.

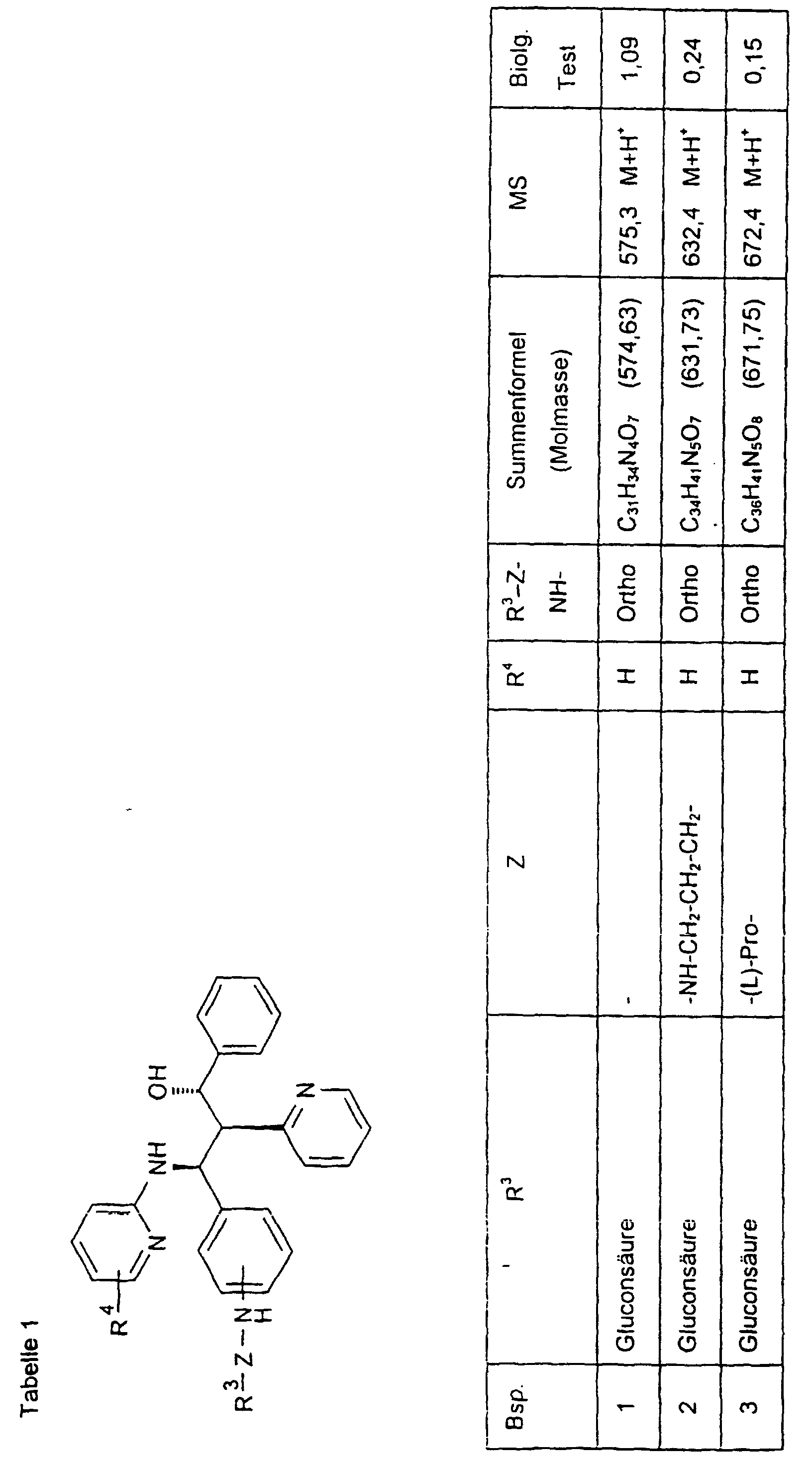

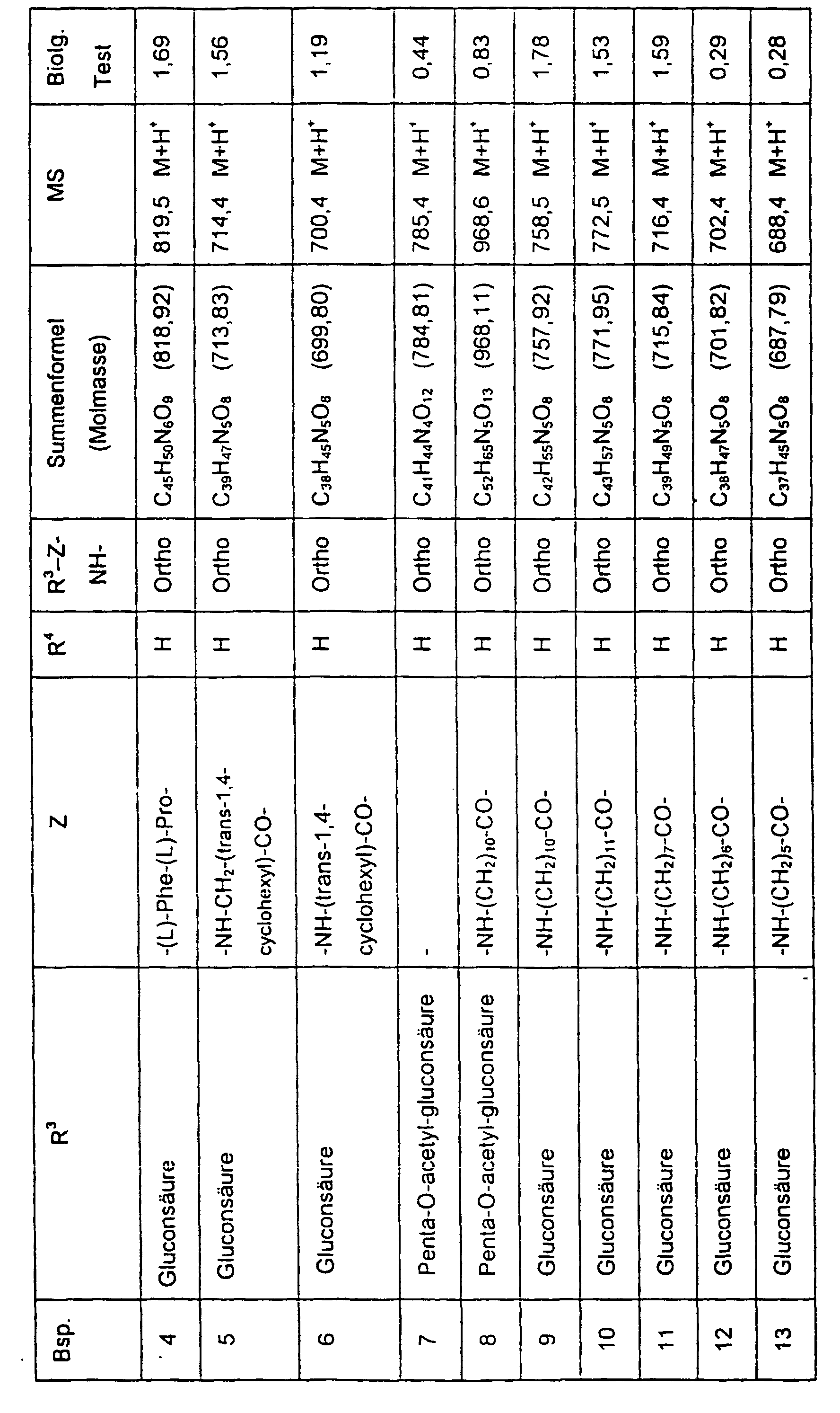

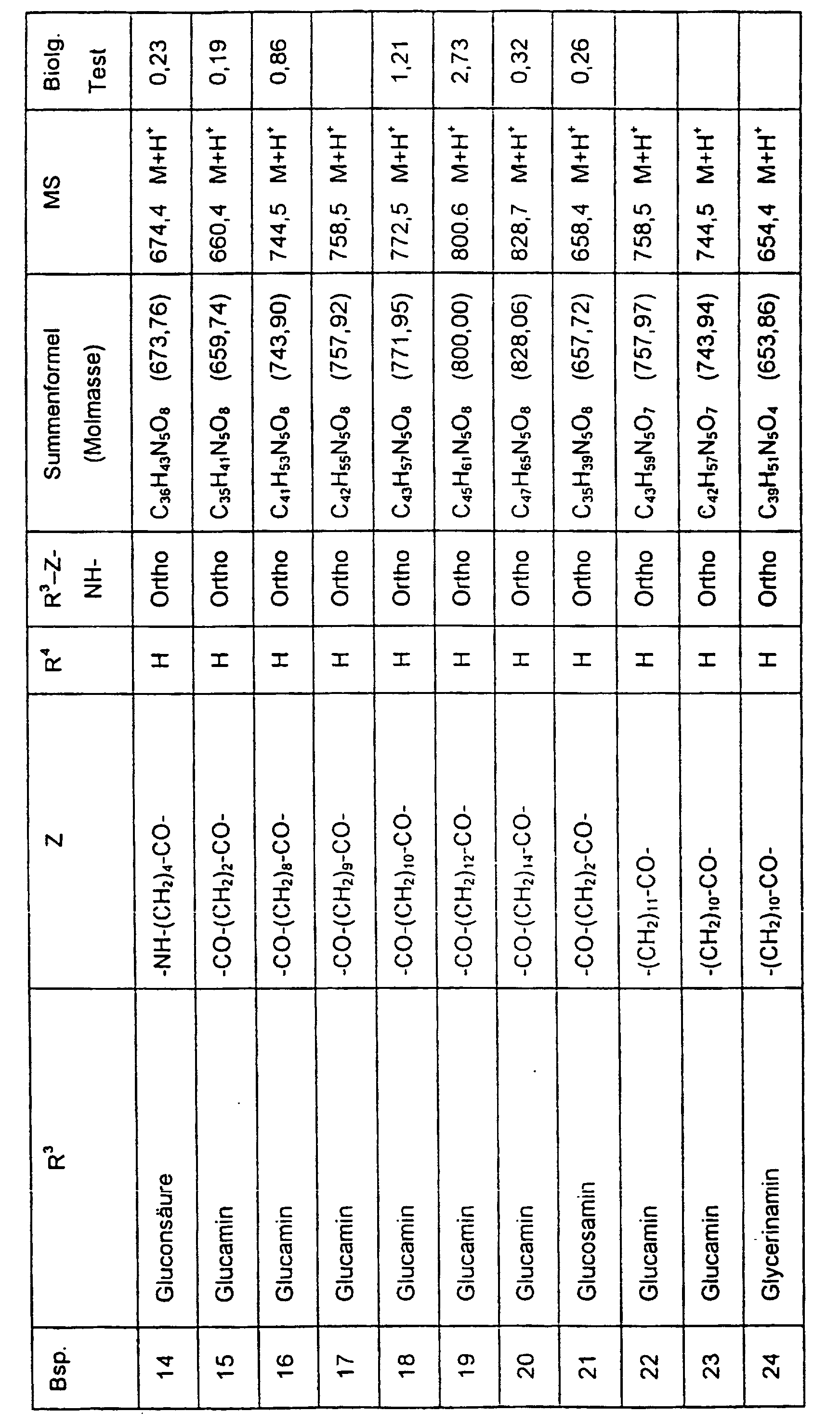

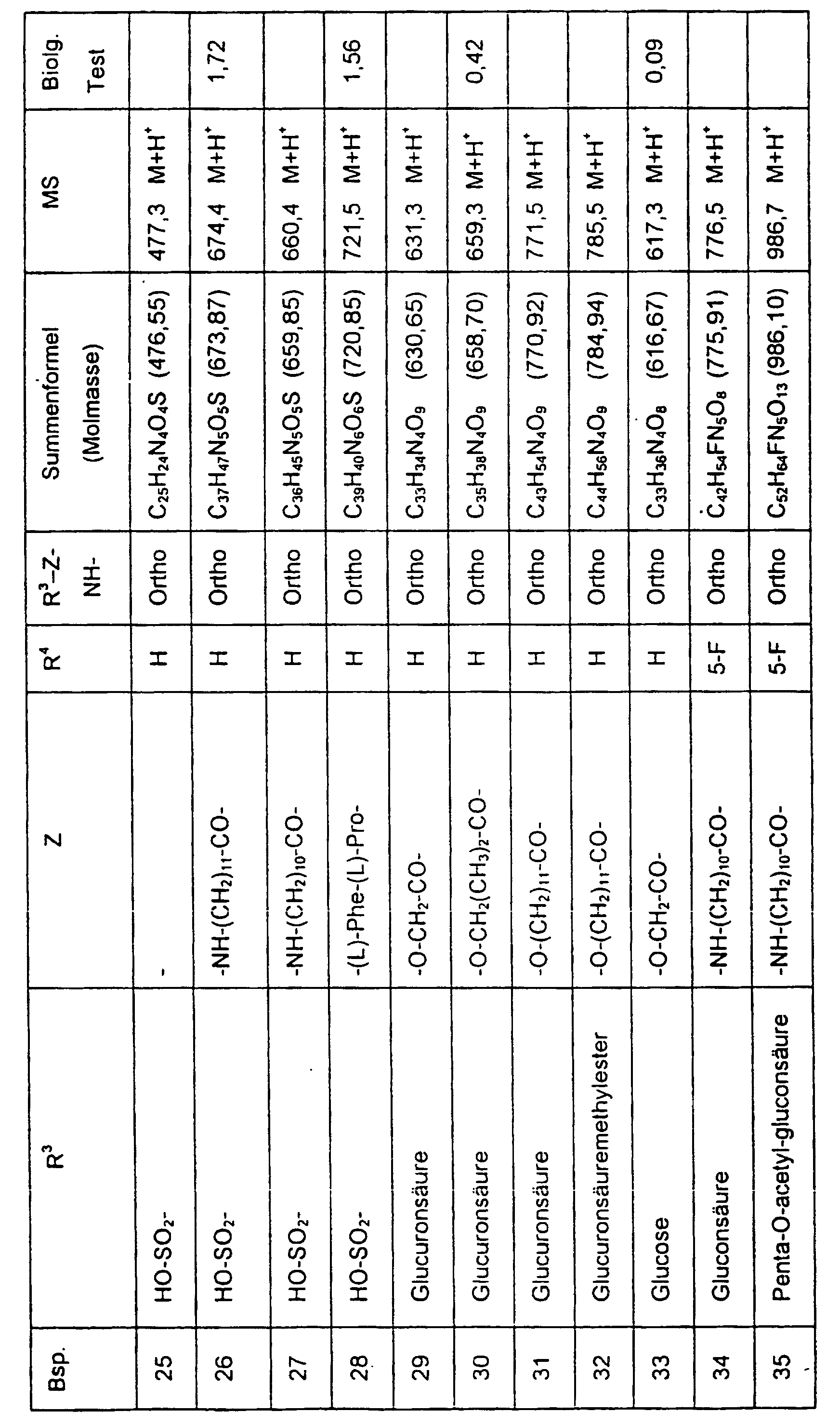

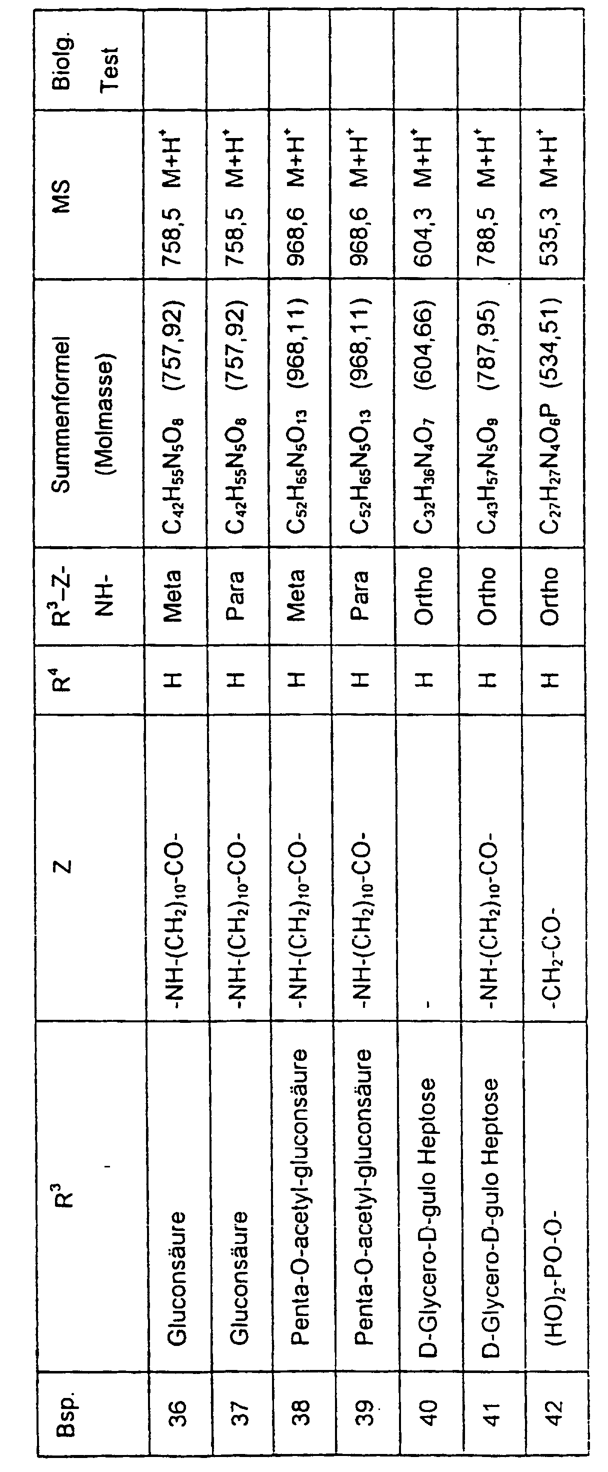

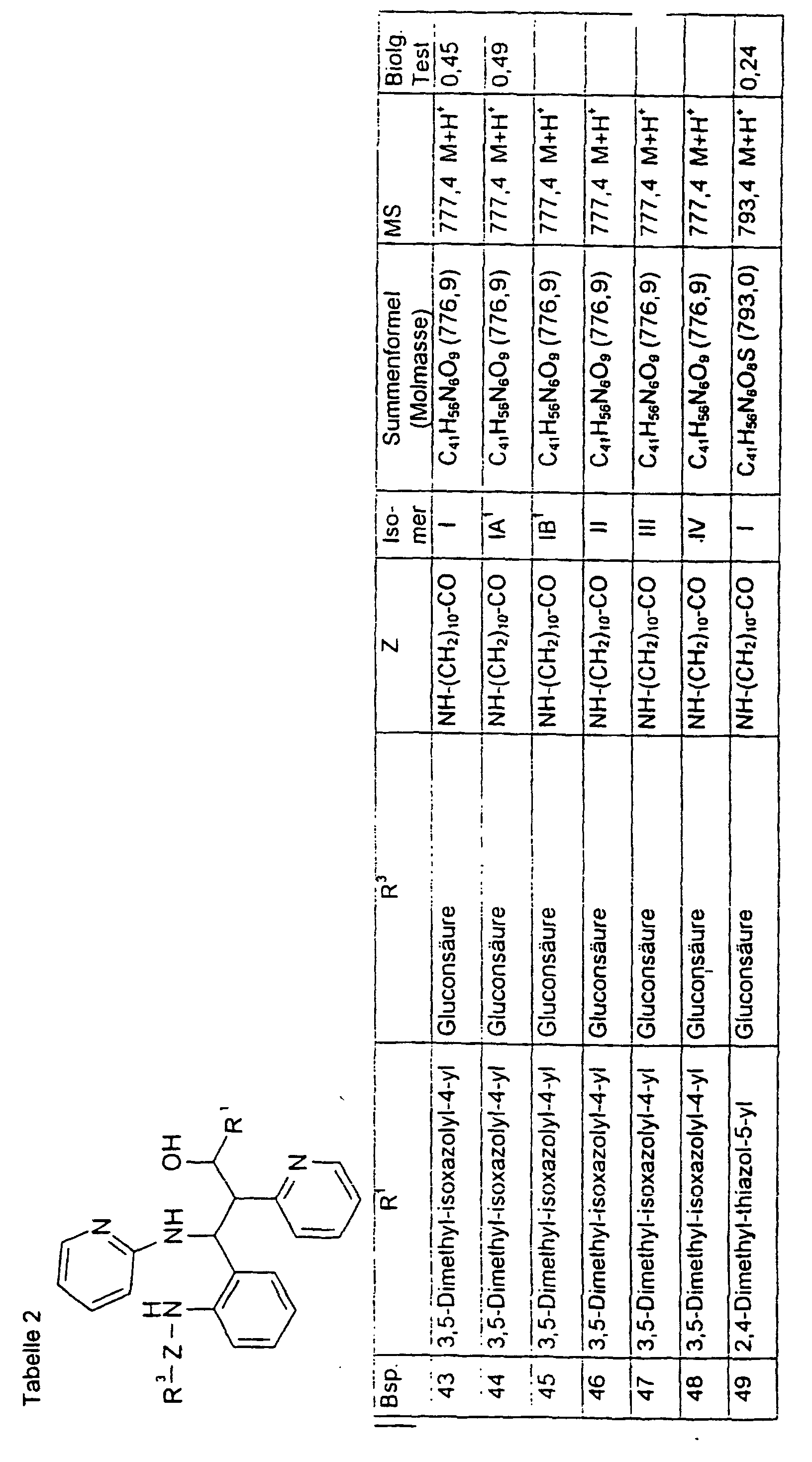

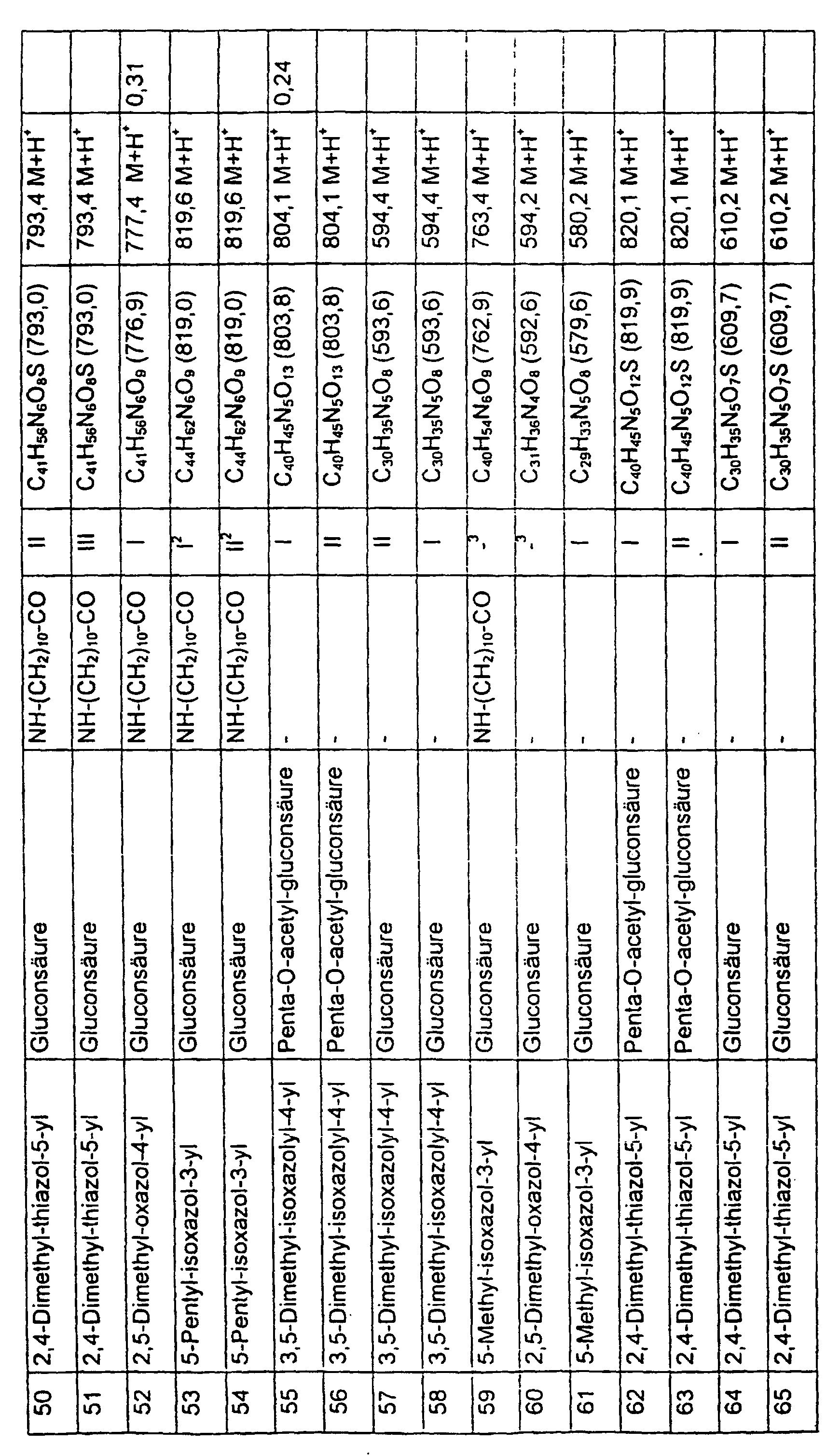

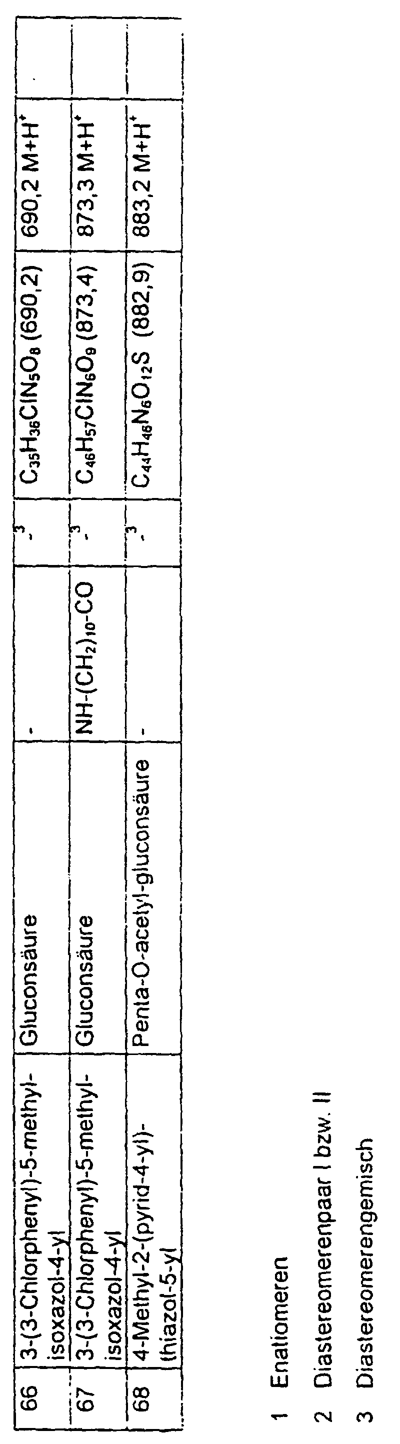

### Test auf Gallensteinbildung

### 1. Prüfsubstanz, Dosis und Applikation

| | mg/kg/d | |
|---|---|---|
| Beispiel 9 der Tabelle 1 (= A1 ) | 100 | 0,1 % im Futter |

### 2. Ziel

In diesem Versuch wurde die Cholesterin-Gallensteinbildung bei gallensteinsensitiven Mäusen untersucht.

### 3. Methodik

### 3.1 Tiermaterial und Haltung

Als Versuchstiere werden männliche C57L Mäuse ( Jackson Laboratories ) mit einem durchschnittlichen Körpergewicht von 25 -30 g zu Adaptionsbeginn verwendet.
Die Tiere werden random in 4 Gruppen eingeteilt (n = 10, Gr. 2 und 3 n = 15). Die Mäuse wurden ab Behandlungsbeginn mit Standard-Nagerfutter von Altromin (Gruppe 1) oder einer lithogenen Diät von Altromin gefüttert (Gruppe 2-4), diese Diät setzt sich wie folgt zusammen:
15 % Butter , 1 % Cholesterin , 50 % Zucker, 20 % Casein, 0,5 % Cholsäure, 5 % Mineralmix , 2,5% Vitamin Mix, 2 % Maisöl ad 100% mit Maisstärke.
Die Tiere werden wöchentlich gewogen, der Futterverbrauch wird durch kontinuierliche Ein- und Auswaage bestimmt und daraus die Dosis berechnet.

### 3.2. Enduntersuchung

Nach 11 bzw. 13 Wochen werden die entsprechenden Tiere jeder Gruppe getötet und anschließend die Gallenblasen präpariert.
Gallensteine in der Gallenblase werden dokumentiert. Die Gallenblase wurde herauspräpariert , das Gewicht wurde ermittelt und die Zusammensetzung der Gallenflüssigkeit und der Gallensteine wurde analysiert.

### 4. Ergebnisse

| Gruppe/Futter | | Dosis [mg/kg/d] | Behandlungszeit [Wochen] | Gallensteinhäufigkeit [n/n] |
|---|---|---|---|---|
| 1 | Normalfutter | - | 13 | 0/10 |
| | 2 Lithogene Diät | - | 11 | 5/15 |
| | 3 Lithogene Diät | - | 13 | 5/15 |
| | 4 Lithogene Diät + Beispiel 9 | 100 | 13 | 0/10 |

Aus den Meßwerten ist abzulesen, daß die erfindungsgemäßen Verbindungen eine Gallensteinbildung wirksam verhindern. Sie eignen sich damit sowohl für die Prophylaxe, als auch zur Behandlung von Gallensteinen.

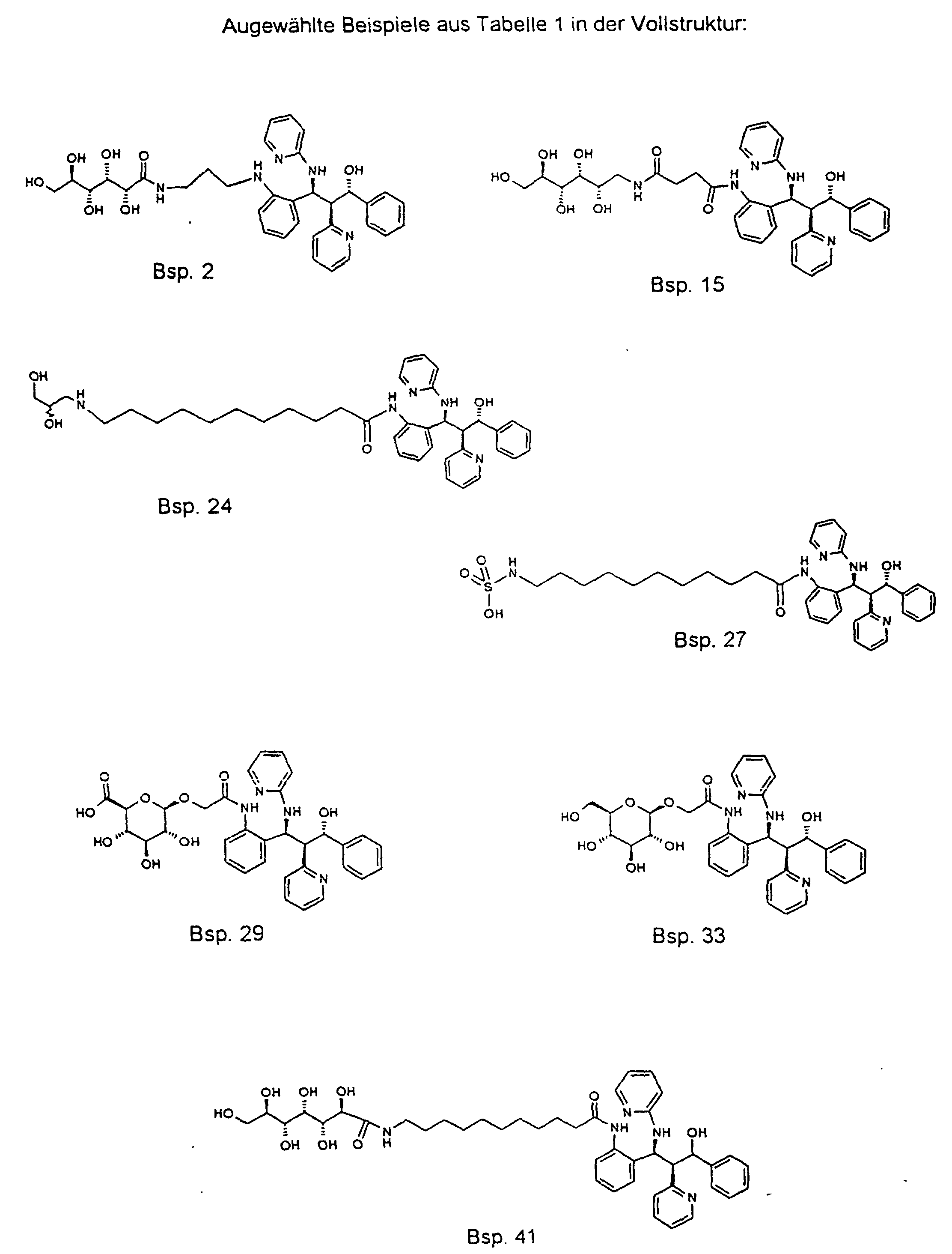

Die nachfolgenden Beispiele dienen zur näheren Erläuterung der Erfindung, ohne dieselbe auf in den Beispielen beschriebene Produkte und Ausführungsformen einzuschränken.

### Beispiel A1 (= Bsp. 9 der Tabelle 1)

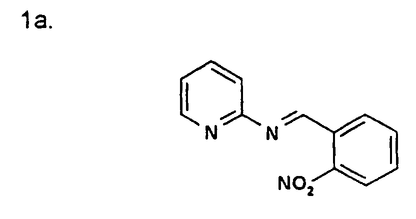

Zu einer Lösung von 25 g (266 mmol) 2-Amino-pyridin und 40 g (265 mmol) 2-Nitrobenzaldehyd in 300 ml Toluol wurden 0,7 g p-Toluolsulfonsäure zugegeben und das Gemisch 6 h unter Rückfluß erhitzt. Nach dem Abkühlen wurde die Hälfte des Lösungsmittels unter Vakuum abgezogen und über Nacht stehengelassen. Der entstandene Niederschlag wurde abgesaugt, mit kaltem Toluol gewaschen und im Vakuum getrocknet. Durch anschließende Umkristallisation aus n-Heptan/Essigsäureethylester 2:1 wurden 48,8 g (81 %) Imin erhalten.
C₁₂H₉N₃O₂ (227,2) MS (FAB) 228,2 M+H⁺

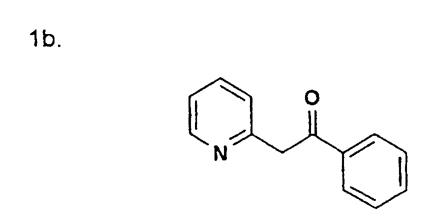

Zu einer Lösung von 50 g (0,54 mol) 2-Picolin in 770 ml Tetrahydrofuran wurden bei - 55°C 250 ml n-Butyllithium (15 % in Hexan) zugetropft und 10 min gerührt. Anschließend wurde auf 0°C erwärmt und nach weiteren 30 min auf -55°C abgekühlt. Danach wurde eine Lösung von 77 g (0,52 mol) N,N-Dimethylbenzamid in 570 ml Tetrahydrofuran langsam zugetropft. Nach der Zugabe wurde auf Zimmertemperatur erwärmt und 1 h gerührt. Nach der Zugabe von 500 ml Wasser und 35 ml konz. HCl wurde die organische Phase abgetrennt und die wäßrige Phase 2 x mit Essigsäureethylester extrahiert. Nach Trocknen über MgSO₄ wurde im Vakuum eingeengt und der Rückstand im Hochvakuum destilliert. Siedepunkt 134-136°C/0,3 mbar. Ausbeute: 47,5 g (47 %) Keton.
C₁₃H₁₁NO (197,2) MS (FAB) 198,1 M+H⁺

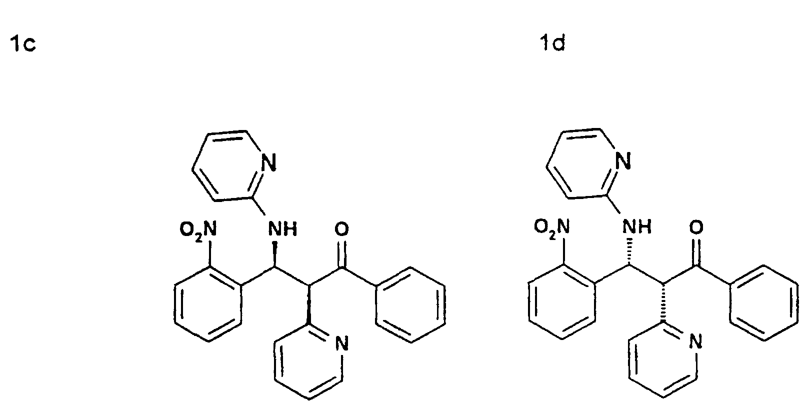

200g (0.89 Mol) Imin aus Beispiel 1 a und 171g (0.88 Mol) Keton aus Beispiel 1 b werden unter Rückfluß in Dichlormethan gelöst. Nachdem die Edukte gelöst sind, läßt man auf Raumtemperatur abkühlen. Die Reaktionslösung wird mit 600 ml Essigsäureethylester und 300 ml n-Heptan verdünnt. Diese Lösung wird mit Hilfe einer Glasfritte (1l), gefüllt mit 500 ml Flashkieselgel, filtriert und mit 500 ml n-Heptan/ Essigsäureethylester (1:2) nachgewaschen. Das Filtrat-wird eingeengt und man erhält 370g Rohprodukt. Das Rohprodukt ist ein Gemisch aus allen vier möglichen Stereoisomeren. Die zwei gewünschten cis-Produkte 1c/d werden durch Kristallisation aus Ethanol erhalten. Dazu wird das Rohprodukt in 600 ml Ethanol gelöst und zwei Tage bei Raumtemperatur stehen gelassen und man erhält 190g Produkt 1c/d. Aus der Mutterlauge kann nach fünf Tagen weitere 106g Produkt 1c/d isoliert werden. Die Stereoisomeren liegen in Lösung in einem Gleichgewicht vor. Das Enatiomerenpaar 1c/1d ist schlecht löslich in Ethanol und kristallisiert aus während das trans-Enatiomempaar in Ethanol löslich ist. Ausbeute 296g (79%) 1c/1d als gelbliche Kristalle.
C₂₅H₂₀N₄O₃ (424,2) MS (FAB) 425.1 M+H⁺

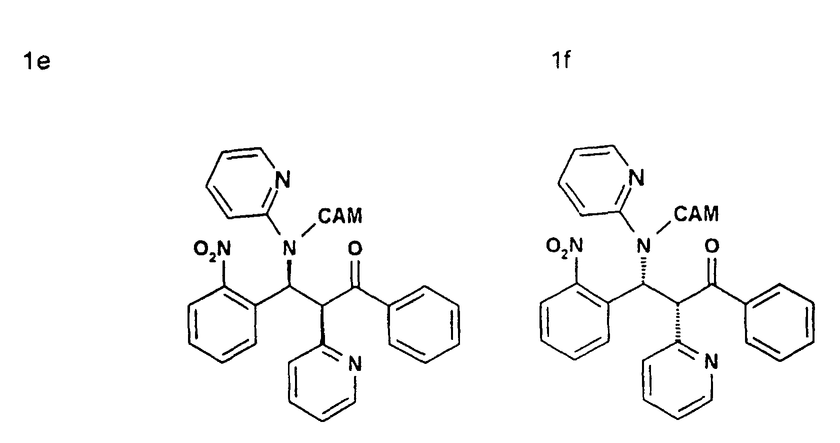

33g (153 mmol) (-)-Camphansäurechlorid CAMCI (Fluka) werden in 500 ml Methylenchlorid gelöst und auf 10°C gekühlt. Zu dieser Lösung werden 50 ml Triethylamin zugegeben. Danach gibt man 52,3 g (123 mmol ) kristallines Keton aus Beispiel 1c/d langsam zu, so daß die Reaktionstemperatur nicht über 20°C steigt. Das Ende der Reaktion wird mit Dünnschichtchromatographie verfolgt (ca. 30 Minuten). Die Reaktionslösung wird mit 500 ml Essigester verdünnt und mit Wasser gewaschen, über MgSO₄ getrocknet, filtriert und eingeengt. Die organische Phase wird aufkonzentriert bis die Kristallisation von 1f beginnt. Dann wird filtriert und man erhält 32,5g (44%) 1f als weißer Feststoff. Die Mutterlauge wird mit 500 ml n-Heptan/Essigester (4:1 ) verdünnt und wieder aufkonzentriert, bis erneut die Kristallisation von Produkt 1e begann. Man erhält 32g (44 %) 1e als farblose Kristalle.
C₃₅H₃₂N₄O₆ (604,7) MS (FAB) 605.3 M+H⁺

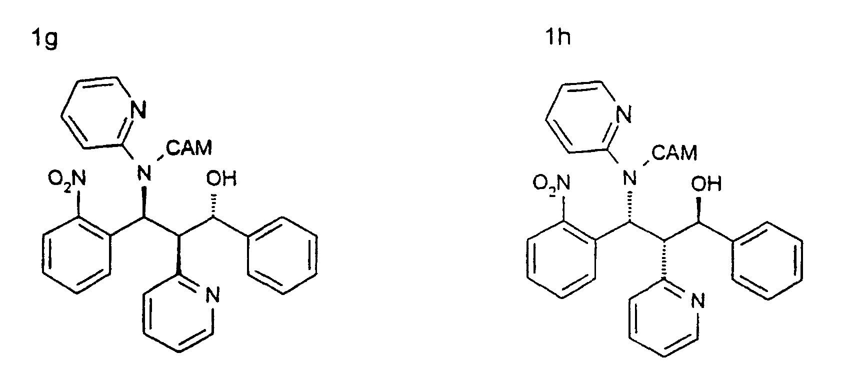

8,5 g (14,1 mmol) Ketoverbindung aus Beispiel 1 e wurden in 150 ml THF/Wasser 10:1 gelöst, mit 2,0 g (53 mmol) Natriumborhydrid versetzt und 10 h bei Zimmertemperatur gerührt. Mit 2 N HCl wurde auf pH 1 gebracht und 30 min bei 50°C gerührt. Nach dem Abkühlen wurde das Reaktionsgemisch mit 2 N NaOH basisch gestellt und 2 x mit Essigester extrahiert. Die organischen Phasen wurden über MgSO₄ getrocknet und soweit eingeengt bis die Kristallisation von 1g beginnt. Ausbeute 3,6 g weiße Kristalle. Die Mutterlauge wird weiter aufkonzentriert und es kann eine zweite Fraktion 1 g (2,45 g) isoliert werden. Die Gesamtausbeute beträgt 6,05 g (71 %) 1g. Mit einer analogen Vorschrift kann 1f umgesetzt werden und man erhält 1h als farblose Kristalle. Die Stereochemie von 1 h konnte durch eine Röntgenstrukturanalyse (Anlage 1) bestimmt werden.
C₃₅H₃₄N₄O₆ (606,7) MS (FAB) 607,3 M+H⁺

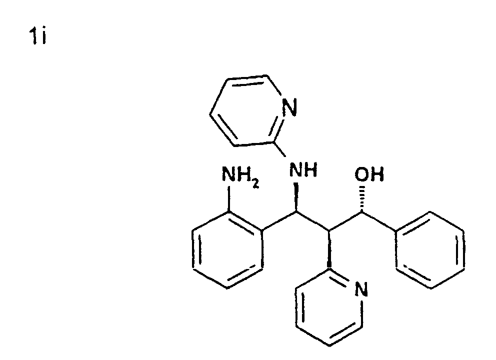

50g KOH werden in 500 ml Ethanol gelöst. In diese Lösung gibt man bei Raumtemperatur 57g (94mmol) Camphansäurederivat 1g, gelöst in 500 ml Methylenchlorid. Nach 2h wird wäßrig aufgearbeitet und man erhält 44,3g Rohprodukt. Dieses Rohprodukt wird in 750 ml Methylenchlorid gelöst und 7,5 g Palladium auf Aktivkohle (10%) zugegeben. Nach 10h Hydrierzeit ist die Wasserstoffaufnahme (rund 6,5 l) beendet. Die Reaktionslösung wird über Kieselgel filtriert und mit 400 ml Methanol nachgewaschen. Nach dem einrotieren des Lösungsmittels erhält man 40g Rohprodukt. Dieses kann in Essigester/n-Heptan umkristallisiert werden und man erhält 25,2 g 1i als farblose Kristalle (68 % über 2 Stufen). Außerdem erhält man aus der Mutterlauge 10g einer amorphen Fraktion von 1i welche 80-90%ige Reinheit aufweist.
C₂₅H₂₄N₄O ( 396,49 ) MS (FAB) 397,2 M+H⁺
Drehwert (α)_{D}²⁰ = +59° (C=1, in CH₂Cl₂)

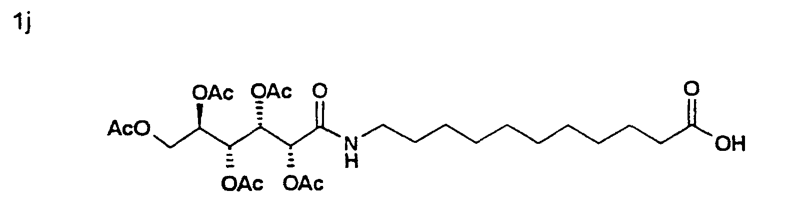

8.0 g (18.8 mmol) Penta-O-acetyl-D-gluconsäurechlorid (Org. Synth. Band 5, 887) werden zu einer Suspension von 8.0 g (40 mmol) 11-Amino-undecansäure (Fluka) in 150 ml wasserfreiem DMF zugegeben. Diese Suspension wird 20 Stunden bei Raumtemperatur kräftig gerührt. Anschließend werden 500 ml Ethylacetat und 200 ml Wasser zugegeben. Die wäßrige Phase wird nochmals mit 250 ml Ethylacetat extrahiert. Die vereinigte organische Phase wird dreimal mit Natriumchloridlösung gewaschen, über MgSO₄ getrocknet, filtriert und eingeengt. Ausbeute 9.5 g (86%) 1j als farbloses ÖI. DC (Methylenchlorid/ Methanol/ konz. Ammoniak 30/10/ 3). R_{f} =0.8.
C₂₇H₄₃NO₁₃ (589.6) MS (FAB) 590,4 M+H⁺

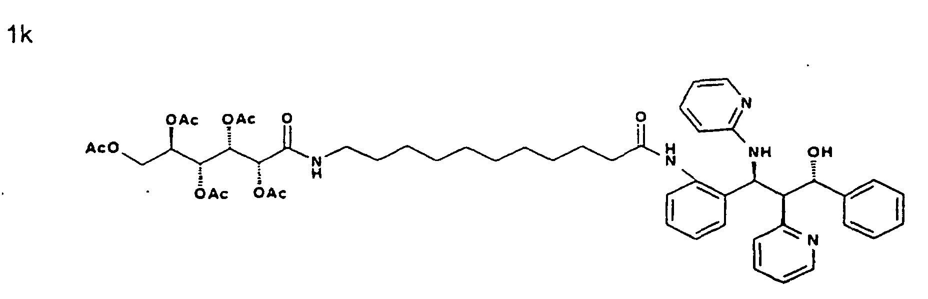

27 g (45,8 mmol) 1j und 16 g (40.3 mmol) 1i werden in 300 ml DMF (Dimethylformamid) gelöst. Nacheinander werden dazu 20 g (61 mmol) TOTU (Fluka), 7 g (50 mmol) Oxim ( Hydroxyimino-cyanessigsäure-ethylester; Fluka) und 17 ml (150 mmol) NEM (4-Ethyl-morpholin) zugegeben. Nach einer Stunde bei Raumtemperatur wird mit 1000 ml Ethylacetat verdünnt und dreimal mit Wasser gewaschen. Die organische Phase wird über MgSO₄ getrocknet, filtriert und eingeengt. Der Rückstand wird mittels Flashchromatographie (Ethylacetat/n-Heptan 1:1) gereinigt und man erhält 37,1 g (95%) 1k als amorpher Feststoff.
C₅₂H₆₅N₅O₁₃ (968,1) MS (FAB) 968,7 M+H⁺

### A1 (= Bsp. 9 der Tabelle 1)

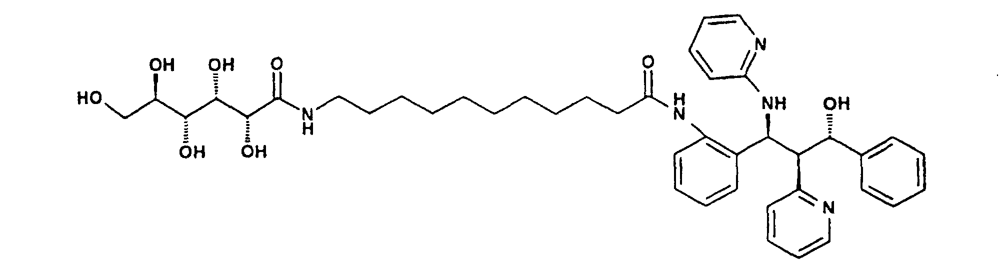

37,1 g (38,3 mmol) 1 k werden in 300 ml Methanol gelöst. Nach Zugabe von 3 ml einer methanolischen 1 M Natriummethanolat- Lösung läßt man eine Stunde bei Raumtemperatur stehen. Dann wird mit methanolischer HCl- Lösung neutralisiert und eingeengt. Der Rückstand wird mit Flashchromatographie (Methylenchlorid/Methanol/ konz. Ammoniak 30/ 5/1) gereinigt und man erhält 24,5 g (84%) A1 (= Bsp. 9 der Tabelle 1) als amorpher Feststoff.
C₄₂H₅₅N₅O₈ (757,9) MS (FAB) 758,4 M+H⁺

### Beispiel A2 (= Bsp. 23 der Tabelle 1)

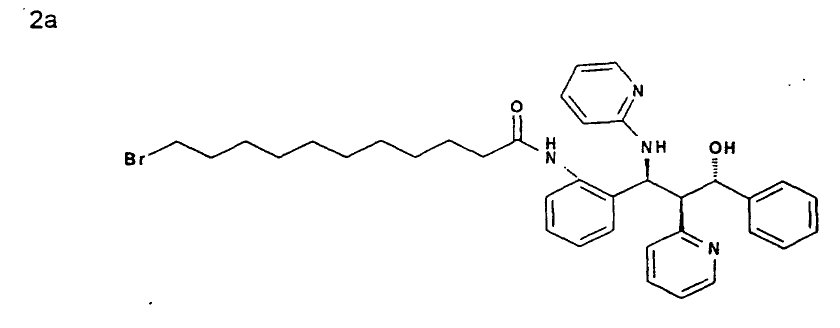

10,0 g (25,0 mmol) 1i und 13,5 g (50,0 mmol) 11-Brom-undecansäure (Fluka) werden in 100 ml DMF (Dimethylformamid) gelöst. Nacheinander werden dazu, bei 0 °C, 15 g (45,7 mmol) TOTU (Fluka) und 17 ml (150 mmol) NEM (4-Ethyl-morpholin) zugegeben. Nach einer Stunde bei 0 °C wird mit 500 ml Ethylacetat verdünnt und dreimal mit Wasser gewaschen. Die organische Phase wird über MgSO₄ getrocknet, filtriert und eingeengt. Der Rückstand wird mittels Flashchromatographie (Ethylacetat/n-Heptan 2:1 ) gereinigt und man erhält 9,9 g (62 %) 2a als amorpher Feststoff.
C₃₆H₄₃BrN₄O₂ (643,7) MS (FAB) 643.3 M+H⁺

### A2 (= Bsp. 23 der Tabelle 1)

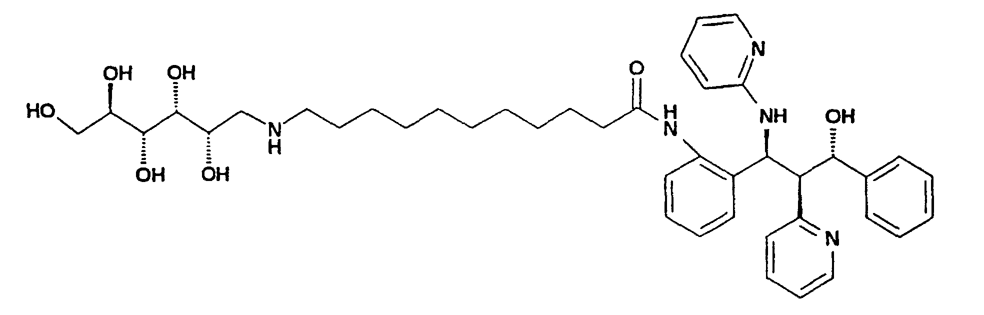

9,87 g (15,3 mmol) 2a werden in 200 ml DMF gelöst. Nach Zugabe von 14 g (77 mmol) Glucamin (Fluka) wird zwei Stunden auf 80°C erwärmt. Danach wird mit 500 ml Ethylacetat verdünnt und dreimal mit Wasser gewaschen. Die organische Phase wird über MgSO₄ getrocknet, filtriert und eingeengt. Der Rückstand wird mittels Flashchromatographie (Methylenchlorid/ Methanol/ konz. Ammoniak 30/ 5/ 1) gereinigt und man erhält 7,3 g (65%) A2 als amorphen Feststoff.
C₄₂H₅₇N₅O₇ (743,9) MS (FAB) 744,4 M+H⁺

### Beispiel A3

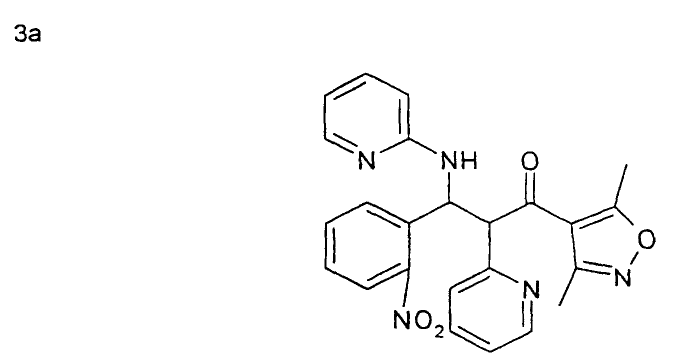

92 g (0,43 mol) 1-(3,5-Dimethyl-isoxazol-4-yl)-2-pyridin-2-yl-ethanon (hergestellt gem. Beispiel 1b, jedoch unter Verwendung von 3,5-Dimethyl-isoxazolylcarbonsäureethylester anstelle von N,N-Dimethylbenzamid) werden in 1200 ml Ethanol heiß gelöst. Bei Zimmertemperatur werden 96,7 g des Imins aus Beispiel 1a zugegeben und das Reaktionsgemisch drei Tage bei Zimmertemperatur gerührt. Nach einigen Stunden beginnt ein gelblicher Feststoff auszufallen. Zur Isolierung des Reaktionsproduktes wird der ausgefallene Feststoff abfiltriert. Man erhält 118,9 g (63%) gelbliche Kristalle vom Schmelzpunkt 139 - 140 °C
C₂₄H₂₁N₅O₄ (443,5) MS (FAB) 444,4 (M+H⁺)

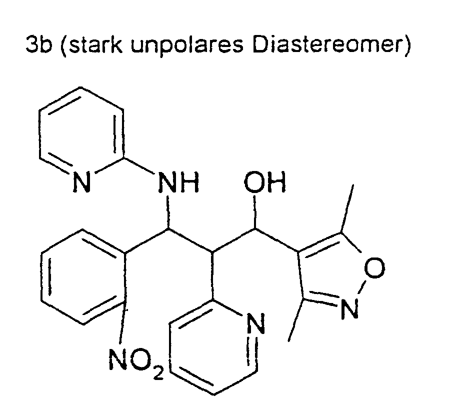

117 g (0,264 mol) des Ketons aus Beispiel 3a werden gemäß des in Beispiel 1g/h beschriebenen Verfahren mittels Natriumborhydrid reduziert. Das erhaltene Rohprodukt wird mittels Chromatographie an Kieselgel unter Verwendung einer Mischung von Ethylacetat/n-Heptan im Verhältnis 2:1 als mobiler Phase gereinigt. Man erhält 76 g (65 %) des stark unpolaren Diastereomeren vom Schmelzpunkt 95 °C; daneben werden noch geringe Mengen der anderen drei möglichen Diastereomeren isoliert.
C₂₄H₂₃N₅O₄ (445,5) MS (FAB) 446,3 (M+H⁺)

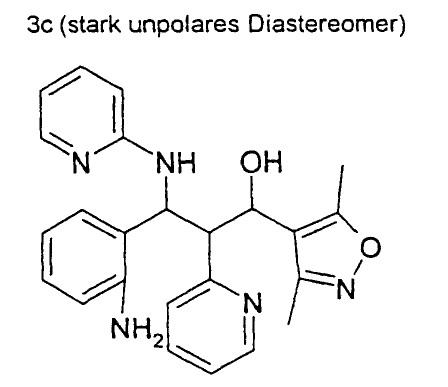

44,5 g (0,1 mol) des Aminopropanols aus Beispiel 3b werden in 1500 ml Ethanol gelöst und bei 20°C mit 570 ml 15%iger wässriger TiCl₃-Lösung versetzt. Nach beendeter Zugabe wird 2,5 Stunden bei Zimmertemperatur gerührt. Zur Aufarbeitung wird die Reaktionsmischung unter reduziertem Druck eingeengt, der verbleibende Rückstand mit Dichlormethan/Wasser extrahiert und mittels Zugabe von NaHCO₃ neutralisiert und unlösliches Titanhydroxid abfiltriert. Nach Trocknung der organischen Phase und Einengen unter reduziertem Druck wird der verbleibende Rückstand über eine kurze Kieselgelsäule filtriert (mobile Phase Ethylacetat). Der nach Entfernen des Elutionsmittels verbleibende Rückstand kristallisiert beim Verrühren mit Diethylether in Form farbloser Kristalle. Man erhält 33,2 g (80%) farblose Kristalle vom Schmelzpunkt 115 °C.
C₂₄H₂₅N₅O₂(415,5) MS (FAB) 416,4 (M+H⁺)

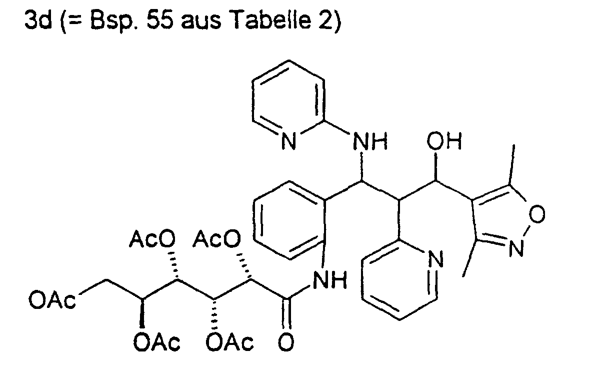

6,2 g (0,015 mol ) der Aminoverbindung aus Beispiel 3c (stark unpolares Diastereomer, welches wiederum als Enatiomerenpaar vorliegt) werden in 100 ml abs. DMF gelöst und unter Rühren mit 6,1 g (0,015 mol) Pentaacetyl-D-Gluconsäure (Org. Synth. Band 5, 887), 5,9 g TOTU (Fluka), 2,1 g Ethylhydroxyiminocyanoacetat und 5,9 ml N-Ethylmorpholin versetzt. Das Reaktionsgemisch wird 20 Stunden bei Zimmertemperatur gerührt. Zur Aufarbeitung wird das Lösungsmittel unter reduziertem Druck entfernt und das erhaltene Rohprodukt mittels Wasser/Dichlormethan unter Verwendung von gesättigter wässriger Natriumbicarbonat-Lösung extrahiert. Nach Trocken der organische Phase mittels Na₂SO₄ und Entfernen des Extraktionsmittels am Rotationsverdampfer wird an Kieselgel chromatographiert (mobile Phase Ethylacetat/n-Heptan = 3:1). Man erhält beide möglichen Diastereomeren in Form farbloser Kristalle:
Unpolares Diastereomer: 3,9 g (31 %) vom Schmelzpunkt 140 °C
C₄₀H₄₅N₅O₁₃ (803,8) MS (FAB) 804,1 (M+H⁺)
Polares Diastereomer: 4,3 g (35 %) vom Schmelzpunkt 204 °C
C₄₀H₄₅N₅O₁₃ (803,8) MS (FAB) 804,1 (M+H⁺)

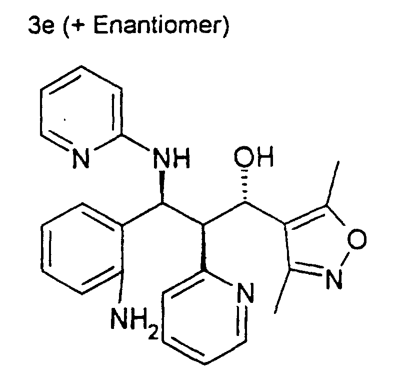

3,5 g (4,4 mmol) des in Beispiel 3d synthetisierten unpolaren Diastereomers werden gemäß Beispiel A1 umgesetzt (Reaktionszeit 1 Stunde; das Reaktionsprodukt ist als Beispiel 58 in Tabelle 2 aufgeführt). Das nach Einengen erhaltene Rohprodukt wird in 80 ml 0,5 M methanolischer HCl gelöst und sechs Stunden bei Zimmertemperatur gerührt. Anschließend wird unter reduziertem Druck eingeengt und mit CH₂Cl₂/Wasser extrahiert. Die organische Phase wird mittels Na₂SO₄ getrocknet und unter reduziertem Druck eingeengt. Man erhält 1,8 g (98 %) farblose Kristalle; die Verbindung liegt in enantiomerenreiner Form vor (Säule CSP-Chiralpak AD, 250x4,6; mobile Phase n-Hexan/Ethanol=10/1; Reinheit 99,7 %; (+)-Enantiomer), was aus Vergleich mit dem Enantiomeren gezeigt werden kann, welches aus analoger Umsetzung des polaren Diastereomeren (Beispiel 3d) resultiert.
C₂₄H₂₅N₅O₂ (415,5) MS (FAB) 416,2 (M+H⁺)

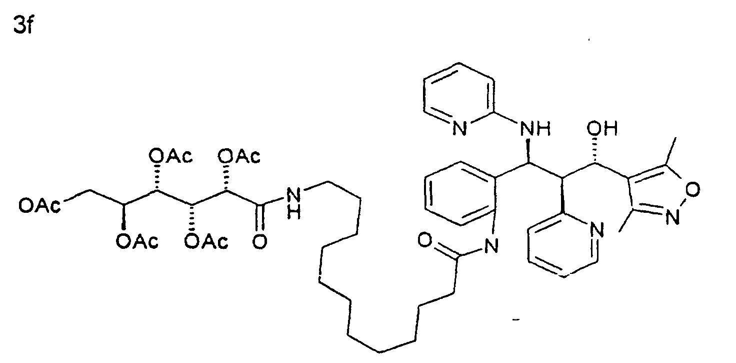

1,8g (4,3 mmol) der enantiomerenreinen Verbindung aus Beispiel 3e werden analog Beispiel 1k mit der Verbindung aus Beispiel 1j umgesetzt. Man erhält 3,7 g (86 %) hellgelbes Öl.
C₅₁H₆₆N₆O₁₄ (987,1) MS (FAB) 987,5 (M+H⁺)

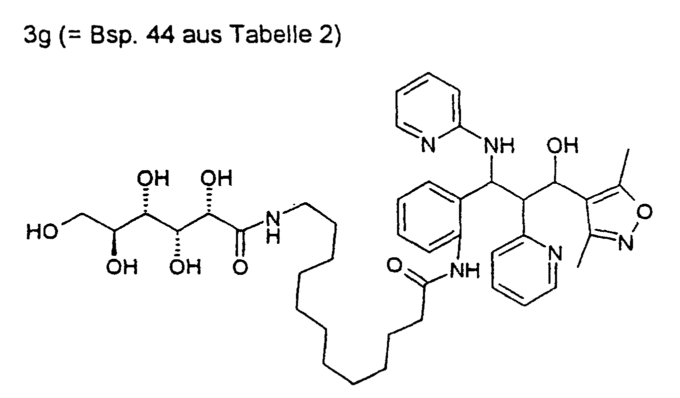

3,7 g (3,8 mmol) der Acetylverbindung aus Beispiel 3f werden gemäß Beispiel A1 deacyliert (Reaktionszeit 2 Stunden) und aufgearbeitet. Man erhält nach Chromatographie an Kieselgel (Laufmittel CH₂Cl₂/Methanol/NH₃[33%] = 30/10/3) 1,78 g (60 %) farblose Kristalle vom Schmelzpunkt 60°C
C₄₁H₅₆N₆O₉ (776,9) MS (FAB) 777,4 (M+H⁺)

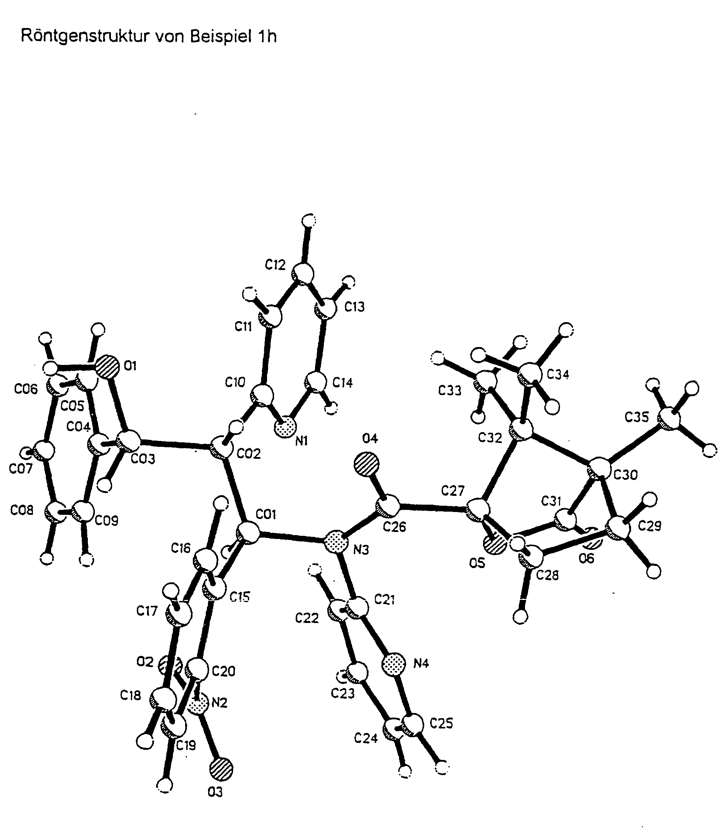

## Patentansprüche

1. Verbindungen der Formel I, 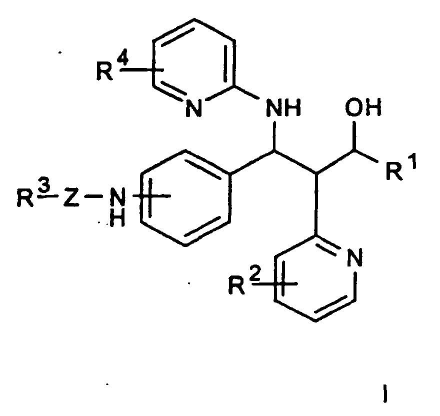 worin bedeuten
R¹ Phenyl, Heteroaryl, unsubstituiert oder gegebenenfalls mit ein bis drei voneinander unabhängigen Resten substituiert wobei der Aromat oder Heteroaromat ein bis dreifach substituiert sein kann mit Fluor, Chlor, Brom, Jod, OH, CF₃, -NO₂, CN, (C₁-C₈)-Alkoxy, (C₁-C₈)-Alkyl, NH₂, -NH-R⁹, -N(R⁹)R¹⁰, CHO, -COOH, -COOR¹¹, -(C=O)-R¹², (C₁-C₆)-alkyl-OH, (C₁-C₆)-alkyl(-OH)-Phenyl, (C₁-C₆)-alkyl-CF₃, (C₁-C₆)-alkyl-NO₂, (C₁-C₆)-alkyl-CN, (C₁-C₆)-alkyl-NH₂, (C₁-C₆)-alkyl-NH-R⁹, (C₁-C₆)-alkyl-N(R⁹)R¹⁰, (C₁-C₆)-alkyl-CHO, (C₁-C₆)-alkyl-COOH, (C₁-C₆)-alkyl-COOR¹¹, (C₁-C₆)-alkyl-(C=O)-R¹², -O-(C₁-C₆)-alkyl-OH, -O-(C₁-C₆)-alkyl-CF₃, -O-(C₁-C₆)-alkyl-NO₂, -O-(C₁-C₆)-alkyl-CN, -O-(C₁-C₆)-alkyl-NH₂, -O-(C₁-C₆)-alkyl-NH-R⁹, -O-(C₁-C₆)-alkyl-N(R⁹)R¹⁰, -O-(C₁-C₆)-alkyl-CHO, -O-(C₁-C₆)-alkyl-COOH, -O-(C₁-C₆)-alkyl-COOR¹¹, -O-(C₁-C₆)-alkyl-(C=O)-R¹², -N-SO₃H, -SO₂-CH₃, -O-(C₁-C₆)-alkyl-O-(C₁-C₆)-alkyl-Phenyl, , (C₁-C₆)-Alkylthio, Pyridyl, wobei in den Alkylresten ein oder mehrere Wasserstoff(e) durch Fluor ersetzt sein können und wobei Phenyl und Pyridyl ihrerseits einfach durch Methyl, Methoxy oder Halogen substituiert sein können;
R² H, OH, CH₂OH, OMe, CHO, NH₂;
R³ Zuckerrest, Dizuckerrest, Trizuckerrest, Tetrazuckerrest, wobei der Zuckerrest, Dizuckerrest, Trizuckerrest oder Tetrazuckerrest gegebenenfalls ein oder mehrfach substituiert ist durch eine Zucker-Schutzgruppe, HO-SO₂-, (HO)₂-PO-;
R⁴ H, Methyl, F, OMe;
R⁹ bis R¹² unabhängig voneinander H, C₁-C₈-Alkyl;
Z - NH-C₀-C₁₆-Alkyl-C=O -, -O-C₀-C₁₆-Alkyl-C=O -,
-(C=O)ₘ-C₁-C₁₆-Alkyl-(C=O)ₙ, Aminosäurerest, Diaminosäurerest,
wobei der Aminosäurerest oder Diaminosäurerest gegebenenfalls ein oder mehrfach substituiert ist durch eine Aminosäure-Schutzgruppe, eine kovalente Bindung;
n 0 oder 1;
m 0 oder 1;
sowie deren pharmazeutisch verträglichen Salze und Ester, die bei Verabreichung in der Lage sind, eine Verbindung der Formel I zu bilden.

2. Verbindungen der Formel I, gemäß Anspruch 1, **dadurch gekennzeichnet, daß** darin bedeuten
R¹ Phenyl, Pyridyl, Thienyl, Furyl, Pyrimidyl, Indolyl, Thiazolyl, Imidazolyl, Coumarinyl, Phthaliminyl, Chinoyl, Piperazinyl, Tetrazolyl, Triazolyl, Oxazolyl Isoxazolyl, Isthiazolyl oder deren benzoannelierte Derivate, wobei der Aromat oder Heteroaromat ein bis dreifach substituiert sein kann mit Fluor, Chlor, Brom, Jod, OH, CF₃, -NO₂, CN, (C₁-C₈)-Alkoxy, (C₁-C₈)-Alkyl, NH₂, -NH-R⁹, -N(R⁹)R¹⁰, CHO, -COOH, -COOR¹¹, -(C=O)-R¹², (C₁-C₆)-alkyl-OH, (C₁-C₆)-alkyl(-OH)-Phenyl, (C₁-C₆)-alkyl-CF₃, (C₁-C₆)-alkyl-NO₂, (C₁-C₆)-alkyl-CN, (C₁-C₆)-alkyl-NH₂, (C₁-C₆)-alkyl-NH-R⁹, (C₁-C₆)-alkyl-N(R⁹)R¹⁰, (C₁-C₆)-alkyl-CHO, (C₁-C₆)-alkyl-COOH, (C₁-C₆)-alkyl-COOR¹¹, (C₁-C₆)-alkyl-(C=O)-R¹², -O-(C₁-C₆)-alkyl-OH, -O-(C₁-C₆)-alkyl-CF₃, -O-(C₁-C₆)-alkyl-NO₂, -O-(C₁-C₆)-alkyl-CN, -O-(C₁-C₆)-alkyl-NH₂, -O-(C₁-C₆)-alkyl-NH-R⁹, -O-(C₁-C₆)-alkyl-N(R⁹)R¹⁰, -O-(C₁-C₆)-alkyl-CHO, -O-(C₁-C₆)-alkyl-COOH, -O-(C₁-C₆)-alkyl-COOR¹¹, -O-(C₁-C₆)-alkyl-(C=O)-R¹², -N-SO₃H, -SO₂-CH₃, -O-(C₁-C₆)-alkyl-O-(C₁-C₆)-alkyl-Phenyl, , (C₁-C₆)-Alkylthio, Pyridyl, wobei in den Alkylresten ein oder mehrere Wasserstoff(e) durch Fluor ersetzt sein können und wobei Phenyl und Pyridyl ihrerseits einfach durch Methyl, Methoxy oder Halogen substituiert sein können;
R² H, OH, CH₂OH, OMe, CHO, NH₂;
R³ Zuckerrest, Dizuckerrest, Trizuckerrest, Tetrazuckerrest, wobei der Zuckerrest, Dizuckerrest, Trizuckerrest oder Tetrazuckerrest gegebenenfalls ein oder mehrfach substituiert ist durch eine der Zucker-Schutzgruppen, HO-SO₂-, (HO)₂-PO-;
R⁴ H, Methyl, F, OMe;
R⁹ bis R¹² unabhängig voneinander H, C₁-C₈-Alkyl;
Z - NH-C₀-C₁₆-Alkyl-C=O -, -O-C₀-C₁₆-Alkyl-C=O-,
- (C=O)ₘ-C₁-C₁₆-Alkyl-(C=O)ₙ, Aminosäurerest, Diaminosäurerest,
wobei der Aminosäurerest oder Diaminosäurerest gegebenenfalls ein oder mehrfach substituiert ist durch eine Aminosäure-Schutzgruppe, eine kovalente Bindung;
n 0 oder 1;
m 0 oder 1;
sowie deren pharmazeutisch verträglichen Salze.

3. Verbindungen der Formel I, gemäß Anspruch 1 oder 2, **dadurch gekennzeichnet, daß** darin bedeuten
R¹ Phenyl, Pyridyl, Thienyl, Furyl, Pyrimidyl, Indolyl, Thiazolyl, Imidazolyl, Coumarinyl, Phthaliminyl, Chinoyl, Piperazinyl, Tetrazolyl, Triazolyl, Oxazolyl Isoxazolyl, Isthiazolyl, wobei der Aromat oder Heteroaromat ein bis zweifach substituiert sein kann mit Fluor, Chlor, Brom, Jod, OH, CF₃, -NO₂, CN, (C₁-C₈)-Alkoxy, (C₁-C₈)-Alkyl, C₃-C₆-Cycloalkyl, NH₂, CHO, -COOH, OCF₃
R² H, OH, CH₂OH, OMe, CHO, NH₂;
R³ Zuckerrest, Dizuckerrest, wobei der Zuckerrest oder Dizuckerrest gegebenenfalls ein oder mehrfach substituiert ist durch eine der Zucker-Schutzgruppen, HO-SO₂-, (HO)₂-PO-;
R⁴ H, Methyl, F, OMe;
Z - NH-C₀-C₁₆-Alkyl-C=O -, -O-C₀-C₁₆-Alkyl-C=O -,
-(C=O)ₘ-C₁-C₁₆-Alkyl-(C=O)ₙ, eine kovalente Bindung;
n 0 oder 1;
m 0 oder 1;
sowie deren physiologisch verträglichen Säureadditionssalze.

4. Verbindungen der Formel I, gemäß einem oder mehreren der Ansprüche 1 bis 3, **dadurch gekennzeichnet, daß** darin bedeuten
R¹ Phenyl, Thiazolyl, Oxazolyl, Isoxazolyl, wobei der Aromat oder Heteroaromat ein bis zweifach substituiert sein kann mit Fluor, Chlor, Brom, (C₁-C₈)-Alkyl;
R² H, OH, CH₂OH, OMe, CHO, NH₂;
R³ 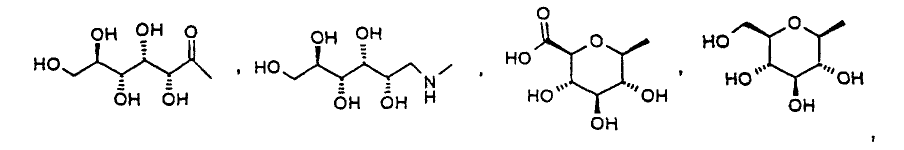 wobei der Zuckerrest gegebenenfalls ein oder mehrfach substituiert ist durch eine der Zucker-Schutzgruppen, HO-SO₂-;
R⁴ H, Methyl, F, OMe;
Z - NH-C₆-C₁₂-Alkyl-C=O -, -O-C₆-C₁₂-Alkyl-C=O -,
-(C=O)ₘ-C₆-C₁₂-Alkyl-(C=O)ₙ;
n 0 oder 1;
m 0 oder 1;
sowie deren physiologisch verträglichen Säureadditionssalze.

5. Arzneimittel enthaltend eine oder mehrere der Verbindungen gemäß einem oder mehreren der Ansprüche 1 bis 4.

6. Arzneimittel enthaltend eine oder mehrere der Verbindungen gemäß einem oder mehreren der Ansprüche 1 bis 4 und ein oder mehrere lipidsenkende Wirkstoffe.

7. Verbindungen gemäß einem oder mehreren der Ansprüche 1 bis 4 zur Anwendung als Medikament zur Prophylaxe oder Behandlung von Lipidstoffwechselstörungen.

8. Verbindungen gemäß einem oder mehreren der Ansprüche 1 bis 4 zur Anwendung als Medikament zur Behandlung von Hyperlipidämie.

9. Verbindungen gemäß einem oder mehreren der Ansprüche 1 bis 4 zur Anwendung als Medikament zur Prophylaxe oder Behandlung von arteriosklerotischer Erscheinungen.

10. Verbindungen gemäß einem oder mehreren der Ansprüche 1 bis 4 in Kombination mit mindestens einem weiteren lipidsenkenden Wirkstoff zur Anwendung als Medikament zur Prophylaxe oder Behandlung von Lipidstoffwechselstörungen.

11. Verbindungen gemäß einem oder mehreren der Ansprüche 1 bis 4 in Kombination mit mindestens einem weiteren lipidsenkenden Wirkstoff als Medikament zur Behandlung von Hyperlipidämie.

12. Verbindungen gemäß einem oder mehreren der Ansprüche 1 bis 4 in Kombination mit mindestens einem weiteren lipidsenkenden Wirkstoff zur Anwendung als Medikament zur Prophylaxe oder Behandlung von arteriosklerotischer Erscheinungen.

13. Verfahren zur Herstellung eines Arzneimittels enthaltend eine oder mehrere der Verbindungen gemäß einem oder mehreren der Ansprüche 1 bis 4, **dadurch gekennzeichnet, daß** der Wirkstoff mit einem pharmazeutisch geeigneten Träger vermischt wird und diese Mischung in eine für die Verabreichung geeignete Form gebracht wird.

14. Verwendung der Verbindungen gemäß einem oder mehreren der Ansprüche 1 bis 4 zur Herstellung eines Medikaments zur Prophylaxe oder Behandlung von Lipidstoffwechselstörungen.

15. Verwendung der Verbindungen gemäß einem oder mehreren der Ansprüche 1 bis 4 zur Herstellung eines Medikaments zur Behandlung von Hyperlipidämie.

## Claims

1. A compound of the formula I 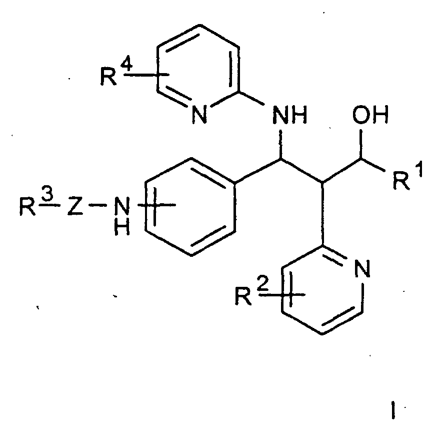 in which
R¹ is phenyl, heteroaryl, unsubstituted or optionally substituted by one to three independent radicals, it being possible for the aromatic or heteroaromatic ring to be mono- to trisubstituted by fluorine, chlorine, bromine, iodine, OH, CF₃, -NO₂, CN, (C₁-C₈)-alkoxy, (C₁-C₈)-alkyl, NH₂, -NH-R⁹, -N(R⁹)R¹⁰, CHO, -COOH, -COOR¹¹, -(C=O)-R¹², (C₁-C₆)-alkyl-OH, (C₁-C₆)-alkyl(-OH)-phenyl, (C₁-C₆)-alkyl-CF₃, (C₁-C₆)-alkyl-NO₂, (C₁-C₆)-alkyl-CN, (C₁-C₆)-alkyl-NH₂, (C₁-C₆)-alkyl-NH-R⁹, (C₁-C₆)-alkyl-N(R⁹)R¹⁰, (C₁-C₆)-alkyl-CHO, (C₁-C₆)-alkyl-COOH, (C₁-C₆)-alkyl-COOR¹¹, (C₁-C₆)-alkyl-(C=O)-R¹², -O-(C₁-C₆)-alkyl-OH, -O-(C₁-C₆)-alkyl-CF₃, -O-(C₁-C₆)-alkyl-NO₂, -O-(C₁-C₆)-alkyl-CN, -O-(C₁-C₆)-alkyl-NH₂, -O-(C₁-C₆)-alkyl-NH-R⁹, -O-(C₁-C₆)-alkyl-N(R⁹)R¹⁰, -O-(C₁-C₆)-alkyl-CHO, -O-(C₁-C₆)-alkyl-COOH, -O-(C₁-C₆)-alkyl-COOR¹¹, -O-(C₁-C₆)-alkyl-(C=O)-R¹², -N-SO₃H, -SO₂-CH₃, -O-(C₁-C₆)-alkyl-O-(C₁-C₆)-alkyl-phenyl, (C₁-C₆)-alkylthio, pyridyl, it being possible for one or more hydrogen(s) in the alkyl radicals to be replaced by fluorine and it being possible for phenyl and pyridyl, in turn, to be monosubstituted by methyl, methoxy or halogen;
R² is H, OH, CH₂OH, OMe, CHO, NH₂;
R³ is a sugar residue, disugar residue, trisugar residue, tetrasugar residue, it being possible for the sugar residue, disugar residue, trisugar residue or tetrasugar residue to be optionally mono- or polysubstituted by a sugar protective group, HO-SO₂-, (HO)₂-PO-;
R⁴ is H, methyl, F, OMe;
R⁹ to R¹² independently of one another are H, C₁-C₈-alkyl;
Z is -NH-C₀-C₁₆-alkyl-C=O -, -O-C₀-C₁₆-alkyl-C=O -,
- (C=O)ₘ-C₁-C₁₆-alkyl-(C=O)ₙ, amino acid residue, diamino acid residue, it being possible for the amino acid residue or diamino acid residue optionally to be mono- or polysubstituted by an amino acid protective group, a covalent bond;
n is 0 or 1;
m is 0 or 1;
or a pharmaceutically tolerated salt or ester thereof which is capable of forming a compound of the formula I upon administration.

2. A compound of the formula I as claimed in claim 1, wherein
R¹ is phenyl, pyridyl, thienyl, furyl, pyrimidyl, indolyl, thiazolyl, imidazolyl, coumarinyl, phthaliminyl, quinoyl, piperazinyl, tetrazolyl, triazolyl, oxazolyl, isoxazolyl, isothiazolyl or their benzo-fused derivatives, it being possible for the aromatic or heteroaromatic ring to be mono- to trisubstituted by fluorine, chlorine, bromine, iodine, OH, CF₃, -NO₂, CN, (C₁-C₈)-alkoxy, (C₁-C₈)-alkyl, NH₂, -NH-R⁹, -N(R⁹)R¹⁰, CHO, -COOH, -COOR¹¹, -(C=O)-R¹², (C₁-C₆)-alkyl-OH, (C₁-C₆)-alkyl(-OH)-phenyl, (C₁-C₆)-alkyl-CF₃, (C₁-C₆)-alkyl-NO₂, (C₁-C₆)-alkyl-CN, (C₁-C₆)-alkyl-NH₂, (C₁-C₆)-alkyl-NH-R⁹, (C₁-C₆)-alkyl-N(R⁹)R¹⁰, (C₁-C₆)-alkyl-CHO, (C₁-C₆)-alkyl-COOH, (C₁-C₆)-alkyl-COOR¹¹, (C₁-C₆)-alkyl-(C=O)-R¹², -O-(C₁-C₆)-alkyl-OH, - O-(C₁-C₆)-alkyl-CF₃, -O-(C₁-C₆)-alkyl-NO₂, -O-(C₁-C₆)-alkyl-CN, -O-(C₁-C₆)-alkyl-NH₂, -O-(C₁-C₆)-alkyl-NH-R⁹, -O-(C₁-C₆)-alkyl-N(R⁹)R¹⁰, -O-(C₁-C₆)-alkyl-CHO, -O-(C₁-C₆)-alkyl-COOH, -O-(C₁-C₆)-alkyl-COOR¹¹, -O-(C₁-C₆)-alkyl-(C=O)-R¹², -N-SO₃H, -SO₂-CH₃, -O-(C₁-C₆)-alkyl-O-(C₁-C₆)-alkyl-phenyl, (C₁-C₆)-alkylthio, pyridyl, it being possible for one or more hydrogen(s) in the alkyl radicals to be replaced by fluorine and it being possible for phenyl and pyridyl, in turn, to be monosubstituted by methyl, methoxy or halogen;
R² is H, OH, CH₂OH, OMe, CHO, NH₂;
R³ is a sugar residue, disugar residue, trisugar residue, tetrasugar residue, it being possible for the sugar residue, disugar residue, trisugar residue or tetrasugar residue to be optionally mono- or polysubstituted by one of the sugar protective groups, HO-SO₂-, (HO)₂-PO-;
R⁴ is H, methyl, F, OMe;
R⁹ to R¹² independently of one another are H, C₁-C₈-alkyl;
Z is -NH-C₀-C₁₆-alkyl-C=O -, -O-C₀-C₁₆-alkyl-C=O -,
-(C=O)ₘ-C₁-C₁₆-alkyl-(C=O)ₙ, amino acid residue, diamino acid residue, it being possible for the amino acid residue or diamino acid residue optionally to be mono- or polysubstituted by an amino acid protective group, a covalent bond;
n is 0 or 1;
or a pharmaceutically tolerated salt thereof.

3. A compound of the formula I as claimed in claim 1 or 2, wherein
R¹ is phenyl, pyridyl, thienyl, furyl, pyrimidyl, indolyl, thiazolyl, imidazolyl, coumarinyl, phthaliminyl, quinoyl, piperazinyl, tetrazolyl, triazolyl, oxazolyl, isoxazolyl, isothiazolyl, it being possible for the aromatic or heteroaromatic ring to be mono- to disubstituted by fluorine, chlorine, bromine, iodine, OH, CF₃, -NO₂, CN, (C₁-C₈)-alkoxy, (C₁-C₈)-alkyl, C₃-C₆-cycloalkyl, NH₂, CHO, -COOH, OCF₃
R² is H, OH, CH₂OH, OMe, CHO, NH₂;
R³ is a sugar residue, disugar residue, the sugar residue or disugar residue optionally being mono- or polysubstituted by one of the sugar protective groups, HO-SO₂-, (HO)₂-PO-;
R⁴ is H, methyl, F, OMe;
Z is -NH-C₀-C₁₆-alkyl-C=O -, -O-C₀-C₁₆-alkyl-C=O -, -(C=O)ₘ-C₁-C₁₆-alkyl-(C=O)ₙ, a covalent bond;
n is 0 or 1;
m is 0 or 1;
or a physiologically tolerated acid addition salt thereof.

4. A compound of the formula I as claimed in one or more of claims 1 to 3, wherein
R¹ is phenyl, thiazolyl, oxazolyl, isoxazolyl, it being possible for the aromatic or heteroaromatic ring to be mono- to disubstituted by fluorine, chlorine, bromine, (C₁-C₈)-alkyl;
R² is H, OH, CH₂OH, OMe, CHO, NH₂;
R³ 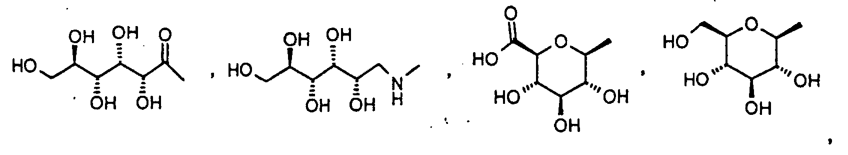 the sugar residue optionally being mono- or polysubstituted by one of the sugar protective groups, HO-SO₂-;
R⁴ is H, methyl, F, OMe;
Z is -NH-C₆-C₁₂-alkyl-C=O -, -O-C₆-C₁₂-alkyl-C=O -,
- (C=O)ₘ-C₆-C₁₂-alkyl-(C=O)ₙ;
n is 0 or 1;
m is 0 or 1;
or a physiologically tolerated acid addition salt thereof.

5. A pharmaceutical comprising one or more of the compounds as claimed in one or more of claims 1 to 4.

6. A pharmaceutical comprising one or more of the compounds as claimed in one or more of claims 1 to 4 and one or more hypolipidemic active substances.

7. A compound as claimed in one or more of claims 1 to 4 for use as medicament for the prophylaxis or treatment of disorders of the lipid metabolism.

8. A compound as claimed in one or more of claims 1 to 4 for use as medicament for the treatment of hyperlipidemia.

9. A compound as claimed in one or more of claims 1 to 4 for use as medicament for the prophylaxis or treatment of arteriosclerotic phenomena.

10. A compound as claimed in one or more of claims 1 to 4 in combination with at least one further hypolipidemic active substance for use as medicament for the prophylaxis or treatment of disorders of the lipid metabolism.

11. A compound as claimed in one or more of claims 1 to 4 in combination with at least one further hypolipidemic active substance as medicament for the treatment of hyperlipidemia.

12. A compound as claimed in one or more of claims 1 to 4 in combination with at least one further hypolipidemic active substance for use as medicament for the prophylaxis or treatment of arteriosclerotic phenomena.

13. A process for the preparation of a pharmaceutical comprising one or more of the compounds as claimed in one or more of claims 1 to 4, which comprises mixing the active substance with a pharmaceutically suitable excipient and bringing this mixture into a form which is suitable for administration.

14. The use of a compound as claimed in one or more of claims 1 to 4 for the preparation of a medicament for the prophylaxis or treatment of disorders of the lipid metabolism.

15. The use of a compound as claimed in one or more of claims 1 to 4 for the preparation of a medicament for the treatment of hyperlipidemia.

## Revendications

1. Composés de formule I, 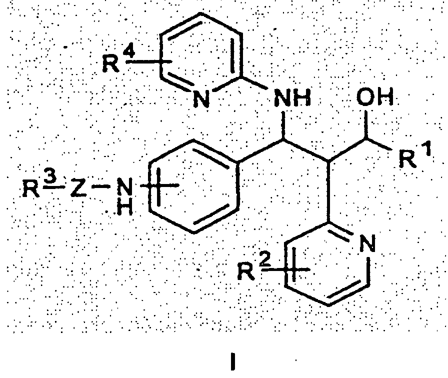 dans laquelle
R¹ représente un phényle, hétéroaryle, non substitué ou éventuellement substitué par un à trois groupes indépendants les uns des autres, le composé aromatique ou hétéroaromatique pouvant être une à trois fois substitué par un fluor, chlore, brome, iode, OH, CF₃, -NO₂, CN, alcoxy en C₁ à C₈, alkyle en C₁ à C₈, NH₂, -NH-R⁹, -N(R⁹)R¹⁰, CHO, -COOH, -COOR¹¹, -(C=O)-R¹², alkyle en C₁ à C₆-OH, alkyle en C₁ à C₆(-OH)-phényle, alkyle en C₁ à C₆-CF₃, alkyle en C₁ à C₆-NO₂, alkyle en C₁ à C₆-CN, alkyle en C₁ à C₆-NH₂, alkyle en C₁ à C₆-NH-R⁹, alkyle en C₁ à C₆-N(R⁹)R¹⁰, alkyle en C₁ à C₆-CHO, alkyle en C₁ à C₆-COOH, alkyle en C₁ à C₆-COOR¹¹, alkyle en C₁ à C₆-(C=O)-R¹², -O-alkyle en C₁ à C₆-OH, -O-alkyle en C₁ à C₆-CF₃, -O-alkyle en C₁ à C₆-NO₂, -O-alkyle en C₁ à C₆-CN, -O-alkyle en C₁ à C₆-NH₂, -O-alkyle en C₁ à C₆-NH-R⁹, -O-alkyle eh C₁ à C₆-N(R⁹)R¹⁰, -O-alkyle en C₁ à C₆-CHO, -O-alkyle en C₁ à C₆-COOH, -O-alkyle en C₁ à C₆-COOR¹¹, -O-alkyle en C₁ à C₆-(C=O)-R¹², -N-SO₃H, -SO₂-CH₃, -O-alkyle en C₁ à C₆-O-alkyle en C₁ à C₆-phényle, alkylthio en C₁ à C₆, pyridyle, dans lesquels dans les groupes alkyle un ou plusieurs hydrogène(s) peuvent être remplacés par des fluors et dans lesquels le phényle et pyridyle de leur côté peuvent être substitués une fois par un méthyle, méthoxy ou halogène ;
R² représente H, OH, CH₂OH, OMe, CHO, NH₂ ;
R³ représente un résidu sucre, un résidu disucre, un résidu trisucre, un résidu tétrasucre, dans lequel le résidu sucre, le résidu disucre, le résidu trisucre ou le résidu tétrasucre est éventuellement substitué une ou plusieurs fois par un groupe de protection de sucre, HO-SO₂-, (HO)₂-PO- ;
R⁴ représente H, un méthyle, F, OMe ;
R⁹ à R¹² représentent indépendamment l'un de l'autre H, un alkyle en C₁ à C₈ ;
Z représente -NH-alkyle en C₀ à C₁₆-C=O-, -O-alkyle en C₀ à C₁₆-C=O-, -(C=O)ₘ-alkyle en C₁ à C₁₆-(C=O)ₙ, un groupe aminoacide, un groupe diaminoacide, dans lesquels le groupe aminoacide ou le groupe diaminoacide est éventuellement une ou plusieurs fois substitué . par un groupe de protection d'aminoacide, une liaison covalente ;
n représente 0 ou 1 ;
m représente 0 ou 1 ;
ainsi que leurs sels et esters pharmaceutiquement acceptables qui sont capables lors de l'administration de former un composé de formule I.

2. Composés de formule I, selon la revendication 1, **caractérisés en ce que**
R¹ représente un phényle, pyridyle, thiényle, furyle, pyrimidyle, indolyle, thiazolyle, imidazolyle, coumarinyle, phtaliminyle, quinoyle, pipérazinyle, tétrazolyle, triazolyle, oxazolyle, isoxazolyle, isthiazolyle ou leurs dérivés accolés à un benzo, dans lesquels le groupe aromatique ou hétéro-aromatique peut être une à trois fois substitué par un fluor, chlore, brome, iode, OH, CF₃, -NO₂, CN, alcoxy en C₁ à C₈, alkyle en C₁ à C₈, NH₂, -NH-R⁹, -N(R⁹)R¹⁰, CHO, -COOH, -COOR¹¹, -(C=O)-R¹², alkyle en C₁ à C₆-OH, alkyle en C₁ à C₆(-OH)-phényle, alkyle en C₁ à C₆-CF₃, alkyle en C₁ à C₆-NO₂, alkyle en C₁ à C₆-CN, alkyle en C₁ à C₆-NH₂, alkyle en C₁ à C₆-NH-R⁹, alkyle en C₁ à C₆-N(R⁹)R¹⁰, alkyle en C₁ à C₆-CHO, alkyle en C₁ à C₆-COOH, alkyle en C₁ à C₆-COOR¹¹, alkyle en C₁ à C₆-(C=O)-R¹², -O-alkyle en C₁ à C₆-OH, -O-alkyle en C₁ à C₆-CF₃, -O-alkyle en C₁ à C₆-NO₂, -O-alkyle en C₁ à C₆-CN, -O-alkyle en C₁ à C₆-NH₂, -O-alkyle en C₁ à C₆-NH-R⁹, -O-alkyle en C₁ à C₆-N(R⁹)R¹⁰, -O-alkyle en C₁ à C₆-CHO, -O-alkyle en C₁ à C₆-COOH, -O-alkyle en C₁ à C₆-COOR¹¹, -O-alkyle en C₁ à C₆-(C=O)-R¹², -N-SO₃H, -SO₂-CH₃, -O-alkyle en C₁ à C₆-O-alkyle en C₁ à C₆-phényle, alkylthio en C₁ à C₆, pyridyle, dans lesquels dans les groupes alkyle un ou plusieurs hydrogène(s) peuvent être remplacés par des fluors et dans lesquels le phényle et pyridyle de leur côté peuvent être substitués une fois par un méthyle, méthoxy ou halogène ;
R² représente H, OH, CH₂OH, OMe, CHO, NH₂ ;
R³ représente un résidu sucre, un résidu disucre, un résidu trisucre, un résidu tétrasucre, dans lequel le résidu sucre, le résidu disucre, le résidu trisucre ou le résidu tétrasucre est éventuellement substitué une ou plusieurs fois par un groupe de protection de sucre, HO-SO₂-, (HO)₂-PO- ;
R⁴ représente H, un méthyle, F, OMe ;
R⁹ à R¹² représentent indépendamment l'un de l'autre H, un alkyle en C₁ à C₈ ;
Z représente -NH-alkyle en C₀ à C₁₆-C=O-, -O-alkyle en C₀ à C₁₆-C=O-, -(C=O)ₘ-alkyle en C₁ à C₁₆-(C=O)ₙ, un groupe aminoacide, un groupe diaminoacide, dans lesquels le groupe aminoacide ou le groupe diaminoacide est éventuellement une ou plusieurs fois substitué par un groupe de protection d'aminoacide, une liaison covalente ;
n représente 0 ou 1 ;
m représente 0 ou 1 ;
ainsi que leurs sels pharmaceutiquement acceptables.

3. Composés de formule I, selon la revendication 1 ou 2, **caractérisés en ce que**
R¹ représente un phényle, pyridyle, thiényle, furyle, pyrimidyle, indolyle, thiazolyle, imidazolyle, coumarinyle, phtaliminyle, quinoyle, pipérazinyle, tétrazolyle, triazolyle, oxazolyle, isoxazolyle, isthiazolyle, dans lesquels le groupe aromatique ou hétéroaromatique peut être une à deux fois substitué par un fluor, chlore, brome, iode, OH, CF₃, -NO₂, CN, alcoxy en C₁ à C₈, alkyle en C₁ à C₈, cycloalkyle en C₃ à C₈, NH₂, CHO, -COOH, OCF₃
R² représente H, OH, CH₂OH, OMe, CHO, NH₂ ;
R³ représente un résidu sucre, un résidu disucre, dans lequel le résidu sucre ou le résidu disucre est éventuellement substitué une ou plusieurs fois par un des groupes de protection des sucres HO-SO₂-, (HO)₂-PO- ;
R⁴ représente H, un méthyle, F, OMe ;
Z représente -NH-alkyle en C₀ à C₁₆-C=O-, -O-alkyle en C₀ à C₁₆-C=O-, -(C=O)ₘ-alkyle en C₁ à C₁₆-(C=O)ₙ, une liaison covalente ;
n représente 0 ou 1 ;
m représente 0 ou 1 ;
ainsi que leurs sels d'addition avec un acide physiologiquement acceptables.

4. Composés de formule I, selon une ou plusieurs des revendications 1 à 3, **caractérisés en ce que**
R¹ représente un phényle, thiazolyle, oxazolyle, isoxazolyle, dans lesquels le groupe aromatique ou hétéroaromatique peut être une à deux fois substitué par un fluor, chlore, brome, alkyle en C₁ à C₈ ;
R² représente H, OH, CH₂OH, OMe, CHO, NH₂ ;
R³ représente 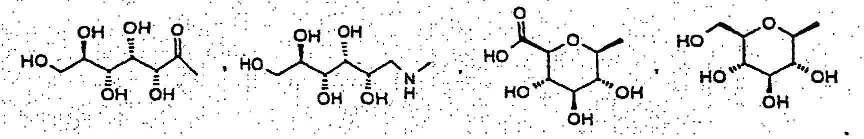 dans lesquels le groupe sucre est éventuellement une ou plusieurs fois substitué par un des groupes de protection des sucres, HO-SO₂- ;
R⁴ représente H, un méthyle, F, OMe ;
Z représente -NH-alkyle en C₆ à C₁₂-C=O-, -O-alkyle en C₆ à C₁₂-C=O-, -(C=O)ₘ-alkyle en C₆ à C₁₂-(C=O)ₙ ;
n représente 0 ou 1 ;
m représente 0 ou 1 ;
ainsi que leurs sels d'addition avec un acide physiologiquement acceptables.

5. Médicament contenant un ou plusieurs des composés selon une ou plusieurs des revendications 1 à 4.

6. Médicament contenant un ou plusieurs des composés selon une ou plusieurs des revendications 1 à 4 et une ou plusieurs substances actives lipidémiantes.

7. Composés selon une ou plusieurs des revendications 1 à 4 pour l'utilisation comme médicament pour la prophylaxie ou le traitement de troubles du métabolisme des lipides.

8. Composés selon une ou plusieurs des revendications 1 à 4 pour l'utilisation comme médicament pour le traitement de l'hyperlipidémie.

9. Composés selon une ou plusieurs des revendications 1 à 4 pour l'utilisation comme médicament pour la prophylaxie ou le traitement de symptômes artériosclérotiques.

10. Composés selon une ou plusieurs des revendications 1 à 4 en combinaison avec au moins une autre substance active lipidémiante pour l'utilisation comme médicament pour la prophylaxie ou le traitement de troubles du métabolisme des lipides.

11. Composés selon une ou plusieurs des revendications 1 à 4 en combinaison avec au moins une autre substance active lipidémiante comme médicament pour le traitement de l'hyperlipidémie.

12. Composés selon une ou plusieurs des revendications 1 à 4 en combinaison avec au moins une autre substance active lipidémiante pour l'utilisation comme médicament pour la prophylaxie ou le traitement de symptômes artériosclérotiques.

13. Procédé pour la préparation d'un médicament contenant un ou plusieurs des composés selon une ou plusieurs des revendications 1 à 4, **caractérisé en ce qu'**on mélange la substance active avec un support pharmaceutique approprié et qu'on met ce mélange sous une forme appropriée à l'administration.

14. Utilisation des composés selon une ou plusieurs des revendications 1 à 4 pour la préparation d'un médicament pour la prophylaxie ou le traitement de troubles du métabolisme des lipides.

15. Utilisation des composés selon une ou plusieurs des revendications 1 à 4 pour la préparation d'un médicament pour le traitement de l'hyperlipidémie.
